# FASCICULE DE BREVET EUROPEEN

(11) **EP 1 981 904 B1**
(45) Date de publication et mention de la délivrance du brevet: **05.04.2017**
(21) Numéro de dépôt: 07730950.8
(22) Date de dépôt: 09.02.2007
(51) Int. Cl.: C07K 14/15

(54) **DOMAINE PEPTIDIQUE NECESSAIRE A L'INTERACTION ENTRE L'ENVELOPPE D'UN VIRUS APPARTENANT AU GROUPE D'INTERFERENCE DE HERV-W ET UN RECEPTEUR HASCT**
PEPTID-DOMÄNE AUS DER HÜLLE EINES HERV-W VIRUS DIE AUSREICHT UM MIT EINEM HASCT-REZEPTOR ZU INTERAGIEREN
PEPTIDE DOMAIN REQUIRED FOR INTERACTION BETWEEN THE ENVELOPE OF A VIRUS PERTAINING TO THE HERV-W INTERFERENCE GROUP AND AN HASCT RECEPTOR

(30) Priorité: 09.02.2006 FR 0650468
(43) Date de publication de la demande: 22.10.2008
(73) Titulaire: Biomerieux S.A., 69280 Marcy l'Etoile (FR)
(72) Inventeur: MALLET, François, 69100 Villeurbanne (FR); ORIOL, Guy, F-42400 Saint Chamond (FR); CHEYNET, Valérie, F-42410 Verin (FR)
(74) Mandataire: Verriest, Philippe
(86) Numéro de dépôt international: PCT/FR2007/000236
(87) Numéro de publication internationale: WO 2007/090967

(56) Documents cités:
- WO-A-2005/080437
- CHEYNET V ET AL: "Synthesis, assembly, and processing of the Env ERVWE1/syncytin human endogenous retroviral envelope." JOURNAL OF VIROLOGY. MAY 2005, vol. 79, no. 9, mai 2005 (2005-05), pages 5585-5593, XP002402067 ISSN: 0022-538X
- LAVILLETTE DIMITRI ET AL: "The envelope glycoprotein of human endogenous retrovirus type W uses a divergent family of amino acid transporters/cell surface receptors" JOURNAL OF VIROLOGY, THE AMERICAN SOCIETY FOR MICROBIOLOGY, US, vol. 76, no. 13, juillet 2002 (2002-07), pages 6442-6452, XP002289561 ISSN: 0022-538X
- CHANG CHINGWEN ET AL: "Functional characterization of the placental fusogenic membrane protein syncytin" BIOLOGY OF REPRODUCTION, SOCIETY FOR THE STUDY OF REPRODUCTION, CHAMPAIGN, IL, US, vol. 71, no. 6, 21 juillet 2004 (2004-07-21), pages 1956-1962, XP002371324 ISSN: 0006-3363
- KIM FELIX J ET AL: "HTLV-1 and -2 envelope SU subdomains and critical determinants in receptor binding" RETROVIROLOGY, BIOMED CENTRAL LTD., LONDON, GB, vol. 1, no. 1, 2 décembre 2004 (2004-12-02), pages 41(1)-41(14), XP021010213 ISSN: 1742-4690
- CHEYNET VALÉRIE ET AL: "Identification of the hASCT2-binding domain of the Env ERVWE1/syncytin-1 fusogenic glycoprotein." RETROVIROLOGY [ELECTRONIC RESOURCE]. 2006, vol. 3, 4 juillet 2006 (2006-07-04), pages 41(1)-41(7), XP002402068 ISSN: 1742-4690

### SEQUENCE LISTING

<110> Japan as Represented by Director-General of National Institute of Infectious Diseases Institute for Antibodies Co., Ltd. Toray Industries, Inc.
<120> Antibody having inhibitory activity against hepatitis C virus infection
<130> PH-6117-PCT
<150> JP 2014-058936
   <151> 2014-03-20
<160> 41
<170> Patent In version 3.5
<210> 1
   <211> 372
   <212> DNA
   <213> Artificial
<220>
   <223> DNA sequence of e2d066 or e2d081 VH region
<220>
   <221> CDS
   <222> (1)..(372)
<400> 1
   cag gtg cag ctg gtg cag tct ggg tct gac gtg aag aag ccc ggg tcc        48
   Gln Val Gln Leu Val Gln Ser Gly Ser Asp Val Lys Lys Pro Gly Ser
   1               5                   10                  15
   tcg gtg agg gtc tcc tgc aag gct tct gga ggc agc ttc aac agt tat        96
   Ser Val Arg Val Ser Cys Lys Ala Ser Gly Gly Ser Phe Asn Ser Tyr
               20                  25                  30
   gct gtg aac tgg gtg cgg cag gcc cct gga cag ggg ctt gaa tgg atg       144
   Ala Val Asn Trp Val Arg Gln Ala Pro Gly Gln Gly Leu Glu Trp Met
           35                  40                  45
   gga agg atc atg cct ttg gtt ggc tta cca gag tac gca gag agg ttc       192
   Gly Arg Ile Met Pro Leu Val Gly Leu Pro Glu Tyr Ala Glu Arg Phe
       50                  55                  60
   caa gag cga gtc acg ttt act gcg gac aaa tcc acg agg aca gcc tac       240
   Gln Glu Arg Val Thr Phe Thr Ala Asp Lys Ser Thr Arg Thr Ala Tyr
   65                  70                  75                  80
   atg gac ctg acc agt ctg agt tct gac gac acg gcc ttt tac ttc tgt       288
   Met Asp Leu Thr Ser Leu Ser Ser Asp Asp Thr Ala Phe Tyr Phe Cys
                   85                  90                  95
   gcg gtc ggt gtt atg aaa att ttt gga gaa gtt cca cta aac ctt gac       336
   Ala Val Gly Val Met Lys Ile Phe Gly Glu Val Pro Leu Asn Leu Asp
               100                 105                 110
   ttc tgg ggt cag ggc acc ctg gtc acc gtc tcg agc                       372
   Phe Trp Gly Gln Gly Thr Leu Val Thr Val Ser Ser
                                       115                 120
<210> 2
   <211> 124
   <212> PRT
   <213> Artificial
<220>
   <223> Amino acid sequence of e2d066 or e2d081 VH région
<400> 2
   Gln Val Gln Leu Val Gln Ser Gly Ser Asp Val Lys Lys Pro Gly Ser
   1               5                   10                  15
   Ser Val Arg Val Ser Cys Lys Ala Ser Gly Gly Ser Phe Asn Ser Tyr
               20                  25                  30
   Ala Val Asn Trp Val Arg Gln Ala Pro Gly Gln Gly Leu Glu Trp Met
           35                  40                  45
   Gly Arg Ile Met Pro Leu Val Gly Leu Pro Glu Tyr Ala Glu Arg Phe
       50                  55                  60
   Gln Glu Arg Val Thr Phe Thr Ala Asp Lys Ser Thr Arg Thr Ala Tyr
   65                  70                  75                  80
   Met Asp Leu Thr Ser Leu Ser Ser Asp Asp Thr Ala Phe Tyr Phe Cys
                   85                  90                  95
   Ala Val Gly Val Met Lys Ile Phe Gly Glu Val Pro Leu Asn Leu Asp
               100                 105                 110
   Phe Trp Gly Gln Gly Thr Leu Val Thr Val Ser Ser
           115                 120
<210> 3
   <211> 330
   <212> DNA
   <213> Artificial
<220>
   <223> DNA sequence of e2d066 VL region
<220>
   <221> CDS
   <222> (1)..(330)
<400> 3
   cag gct gtg ctc act cag ccg tct tcc gtg tct ggg tct cct gga cag       48
   Gln Ala Val Leu Thr Gln Pro Ser Ser Val Ser Gly Ser Pro Gly Gln
   1               5                   10                  15
   tcg atc acc atc tcc tgc act gga acc agc aat gac gtg ggt agt tat          96
   Ser Ile Thr Ile Ser Cys Thr Gly Thr Ser Asn Asp Val Gly Ser Tyr
               20                  25                  30
   cgc tat gtc tcc tgg ttc caa cag cac cca ggc aaa gcc ccc aaa ctc         144
   Arg Tyr Val Ser Trp Phe Gln Gln His Pro Gly Lys Ala Pro Lys Leu
           35                  40                  45
   atg att tat gat gtc aat aaa cgg ccc tca ggg gtt tct agt cgc ttc         192
   Met Ile Tyr Asp Val Asn Lys Arg Pro Ser Gly Val Ser Ser Arg Phe
       50                  55                  60
   tct ggc tcc aag tct ggc aac acg gcc tcc ctg acc atc tct ggg ctc         240
   Ser Gly Ser Lys Ser Gly Asn Thr Ala Ser Leu Thr Ile Ser Gly Leu
   65                  70                  75                  80
   cag gct gag gac gag gct aat tat tac tgc agc tca tat aca aga agc         288
   Gln Ala Glu Asp Glu Ala Asn Tyr Tyr Cys Ser Ser Tyr Thr Arg Ser
                   85                  90                  95
   agc agt ttg gct ttc ggc gga ggg acc aaa ctg acc gtc cta                 330
   Ser Ser Leu Ala Phe Gly Gly Gly Thr Lys Leu Thr Val Leu
               100                 105                 110
<210> 4
   <211> 110
   <212> PRT
   <213> Artificial
<220>
   <223> Amino acid sequence of e2d066 VL region
<400> 4
   Gln Ala Val Leu Thr Gln Pro Ser Ser Val Ser Gly Ser Pro Gly Gln
   1               5                   10                  15
   Ser Ile Thr Ile Ser Cys Thr Gly Thr Ser Asn Asp Val Gly Ser Tyr
               20                  25                  30
   Arg Tyr Val Ser Trp Phe Gln Gln His Pro Gly Lys Ala Pro Lys Leu
           35                  40                  45
   Met Ile Tyr Asp Val Asn Lys Arg Pro Ser Gly Val Ser Ser Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asn Tyr Tyr Cys Ser Ser Tyr Thr Arg Ser
                   85                  90                  95
   Ser Ser Leu Ala Phe Gly Gly Gly Thr Lys Leu Thr Val Leu
               100                 105                 110
<210> 5
   <211> 5
   <212> PRT
   <213> Artificial
<220>
   <223> Amino acid sequence of e2d066 or e2d081 VH CDR1
<400> 5
   Ser Tyr Ala Val Asn
   1               5
<210> 6
   <211> 17
   <212> PRT
   <213> Artificial
<220>
   <223> Amino acid sequence of e2d066 or e2d081 VH CDR2
<400> 6 Glu
<210> 7
   <211> 15
   <212> PRT
   <213> Artificial
<220>
   <223> Amino acid sequence of e2d066 or e2d081 VH CDR3
<400> 7
   Gly Val Met Lys Ile Phe Gly Glu Val Pro Leu Asn Leu Asp Phe
   1               5                   10                  15
<210> 8
   <211> 14
   <212> PRT
   <213> Artificial
<220>
   <223> Amino acid sequence of e2d066 VL CDR1
<400> 8
   Thr Gly Thr Ser Asn Asp Val Gly Ser Tyr Arg Tyr Val Ser
   1               5                   10
<210> 9
   <211> 7
   <212> PRT
   <213> Artificial
<220>
   <223> Amino acid sequence of e2d066 VL CDR2
<400> 9
   Asp Val Asn Lys Arg Pro Ser
   1               5
<210> 10
   <211> 10
   <212> PRT
   <213> Artificial
<220>
   <223> Amino acid sequence of e2d066 VL CDR3
<400> 10
   Ser Ser Tyr Thr Arg Ser Ser Ser Leu Ala
   1               5                   10
<210> 11
   <211> 378
   <212> DNA
   <213> Artificial
<220>
   <223> DNA sequence of e2d073 VH region
<220>
   <221> CDS
   <222> (1)..(378)
<400> 11
   cag gtg cag ctg gtg cag tct ggg gct gag gtg aag aag cct ggg tcc       48
   Gln Val Gln Leu Val Gln Ser Gly Ala Glu Val Lys Lys Pro Gly Ser
   1               5                   10                  15
   tcg gtg aag gtc tcc tgc aag gct tct gga gcc tcc ttc aat agc ttt       96
   Ser Val Lys Val Ser Cys Lys Ala Ser Gly Ala Ser Phe Asn Ser Phe
               20                  25                  30
   gct atc gac tgg gtg cga cag gcc cct gga caa ggg ctt gag tgg atg      144
   Ala Ile Asp Trp Val Arg Gln Ala Pro Gly Gln Gly Leu Glu Trp Met
           35                  40                  45
   gga agg atc atc ccc atc gca gat gtt tca gac tac gca cag aag ttc      192
   Gly Arg Ile Ile Pro Ile Ala Asp Val Ser Asp Tyr Ala Gln Lys Phe
       50                  55                  60
   cag ggc aga gtc aca att acc gcg gac aaa tcc acg agg aca gcc tac      240
   Gln Gly Arg Val Thr Ile Thr Ala Asp Lys Ser Thr Arg Thr Ala Tyr
   65                  70                  75                  80
   atg gag ctg acc acc ctg aga tct gac gac acg gcc gta tat tac tgt      288
   Met Glu Leu Thr Thr Leu Arg Ser Asp Asp Thr Ala Val Tyr Tyr Cys
                   85                  90                  95
   gcg acc tcc cct atg ctt acg ttt ggg gga cct aac gct ttt ggt gct        336
   Ala Thr Ser Pro Met Leu Thr Phe Gly Gly Pro Asn Ala Phe Gly Ala
               100                 105                 110
   ttt gat gtc tgg ggc cag ggg aca atg gtc acc gtc tcg agc                378
   Phe Asp Val Trp Gly Gln Gly Thr Met Val Thr Val Ser Ser
           115                 120                 125
<210> 12
   <211> 126
   <212> PRT
   <213> Artificial
<220>
   <223> Amino acid sequence of e2d073 VH region
<400> 12
   Gln Val Gln Leu Val Gln Ser Gly Ala Glu Val Lys Lys Pro Gly Ser
   1               5                   10                  15
   Ser Val Lys Val Ser Cys Lys Ala Ser Gly Ala Ser Phe Asn Ser Phe
               20                  25                  30
   Ala Ile Asp Trp Val Arg Gln Ala Pro Gly Gln Gly Leu Glu Trp Met
           35                  40                  45
   Gly Arg Ile Ile Pro Ile Ala Asp Val Ser Asp Tyr Ala Gln Lys Phe
       50                  55                  60
   Gln Gly Arg Val Thr Ile Thr Ala Asp Lys Ser Thr Arg Thr Ala Tyr
   65                  70                  75                  80
   Met Glu Leu Thr Thr Leu Arg Ser Asp Asp Thr Ala Val Tyr Tyr Cys
                   85                  90                  95
   Ala Thr Ser Pro Met Leu Thr Phe Gly Gly Pro Asn Ala Phe Gly Ala
               100                 105                 110
   Phe Asp Val Trp Gly Gln Gly Thr Met Val Thr Val Ser Ser
           115                 120                 125
<210> 13
   <211> 324
   <212> DNA
   <213> Artificial
<220>
   <223> DNA sequence of e2d073 VL region
<220>
   <221> CDS
   <222> (1)..(324)
<400> 13
   gac atc cag atg acc cag tct cca tcc tcc ctg tcc gca tct gta gga        48
   Asp Ile Gln Met Thr Gln Ser Pro Ser Ser Leu Ser Ala Ser Val Gly
   1               5                   10                  15
   gac aga gtc acc atc act tgc cgg gca agt cag agc att agc agc tat        96
   Asp Arg Val Thr Ile Thr Cys Arg Ala Ser Gln Ser Ile Ser Ser Tyr
               20                  25                  30
   tta aat tgg tat cag cag aaa cca ggg aaa gcc cct aag ctc ctg atc       144
   Leu Asn Trp Tyr Gln Gln Lys Pro Gly Lys Ala Pro Lys Leu Leu Ile
           35                  40                  45
   tat gct gca tcc agt ttg caa agt ggg gtc cca tca agg ttc agt ggc       192
   Tyr Ala Ala Ser Ser Leu Gln Ser Gly Val Pro Ser Arg Phe Ser Gly
       50                  55                  60
   agt gga tct ggg aca gat ttc act ctc acc gtc agc agt ctg caa cct       240
   Ser Gly Ser Gly Thr Asp Phe Thr Leu Thr Val Ser Ser Leu Gln Pro
   65                  70                  75                  80
   gaa gat ttt gca act tac tac tgt caa cag agt tac agt acc cct caa       288
   Glu Asp Phe Ala Thr Tyr Tyr Cys Gln Gln Ser Tyr Ser Thr Pro Gln
                   85                  90                  95
   ttc act ttc ggc cct ggg acc aaa gtg gat atc aaa                       324
   Phe Thr Phe Gly Pro Gly Thr Lys Val Asp Ile Lys
               100                 105
<210> 14
   <211> 108
   <212> PRT
   <213> Artificial
<220>
   <223> Amino acid sequence of e2d073 VL region
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Val Ser Ser Leu Gln Pro
   65                  70                  75                  80
   Glu Asp Phe Ala Thr Tyr Tyr Cys Gln Gln Ser Tyr Ser Thr Pro Gln
                   85                  90                  95
   Phe Thr Phe Gly Pro Gly Thr Lys Val Asp Ile Lys
               100                 105
<210> 15
   <211> 5
   <212> PRT
   <213> Artificial
<220>
   <223> Amino acid sequence of e2d073 VH CDR1
<400> 15
   Ser Phe Ala Ile Asp
   1               5
<210> 16
   <211> 17
   <212> PRT
   <213> Artificial
<220>
   <223> Amino acid sequence of e2d073 VH CDR2
<400> 16
   Arg Ile Ile Pro Ile Ala Asp Val Ser Asp Tyr Ala Gln Lys Phe Gln
   1               5                   10                  15
   Gly
<210> 17
   <211> 17
   <212> PRT
   <213> Artificial
<220>
   <223> Amino acid sequence of e2d073 VH CDR3
<400> 17 Val
<210> 18
   <211> 11
   <212> PRT
   <213> Artificial
<220>
   <223> Amino acid sequence of e2d073 VL CDR1
<400> 18
   Arg Ala Ser Gln Ser Ile Ser Ser Tyr Leu Asn
   1               5                   10
<210> 19
   <211> 7
   <212> PRT
   <213> Artificial
<220>
   <223> Amino acid sequence of e2d073 VL CDR2
<400> 19
   Ala Ala Ser Ser Leu Gln Ser
   1               5
<210> 20
   <211> 10
   <212> PRT
   <213> Artificial
<220>
   <223> Amino acid sequence of e2d073 VL CDR3
<400> 20
   Gln Gln Ser Tyr Ser Thr Pro Gln Phe Thr
   1               5                   10
<210> 21
   <211> 330
   <212> DNA
   <213> Artificial
<220>
   <223> DNA sequence of e2d081 VL region
<400> 21
   cagtctgtgt tgacgcagcc gccctcagtg tctgcggccc caggacagaa ggtcaccatc      60
   tcctgctctg gaaccagctc caacattggg gataattatg tatcctggta ccagcatctc     120
   ccaggatcag cccccaaact cctcatttat gacaataata agcgaccctc agggattcct     180
   gaccgattct ctggctccaa gtctggcacg tcagccaccc tgggcatcac cggactccag     240
   actggagacg aggccgattt ttactgcgga acatgggata gcagcctgag ttctgtggtg     300
   ttcggcggag ggaccaagct gaccgtccta                                      330
<210> 22
   <211> 110
   <212> PRT
   <213> Artificial
<220>
   <223> Amino acid sequence of e2d081 VL region
<400> 22
   Gln Ser Val Leu Thr Gln Pro Pro Ser Val Ser Ala Ala Pro Gly Gln
   1               5                   10                  15
   Lys Val Thr Ile Ser Cys Ser Gly Thr Ser Ser Asn Ile Gly Asp Asn
               20                  25                  30
   Tyr Val Ser Trp Tyr Gln His Leu Pro Gly Ser Ala Pro Lys Leu Leu
           35                  40                  45
   Ile Tyr Asp Asn Asn Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Phe Tyr Cys Gly Thr Trp Asp Ser Ser Leu
                   85                  90                  95
   Ser Ser Val Val Phe Gly Gly Gly Thr Lys Leu Thr Val Leu
               100                 105                 110
<210> 23
   <211> 13
   <212> PRT
   <213> Artificial
<220>
   <223> Amino acid sequence of e2d081 VL CDR1
<400> 23
   Ser Gly Thr Ser Ser Asn Ile Gly Asp Asn Tyr Val Ser
   1 5 10
<210> 24
   <211> 7
   <212> PRT
   <213> Artificial
<220>
   <223> Amino acid sequence of e2d081 VL CDR2
<400> 24
   Asp Asn Asn Lys Arg Pro Ser
   1 5
<210> 25
   <211> 11
   <212> PRT
   <213> Artificial
<220>
   <223> Amino acid sequence of e2d081 VL CDR3
<400> 25
   Gly Thr Trp Asp Ser Ser Leu Ser Ser Val Val
   1 5 10
<210> 26
   <211> 454
   <212> PRT
   <213> Artificial
<220>
   <223> Amino acid sequence of entire heavy chain of e2d066 IgG antibody
<400> 26
   Gln Val Gln Leu Val Gln Ser Gly Ser Asp Val Lys Lys Pro Gly Ser
   1               5                   10                  15
   Ser Val Arg Val Ser Cys Lys Ala Ser Gly Gly Ser Phe Asn Ser Tyr
               20                  25                  30
   Ala Val Asn Trp Val Arg Gln Ala Pro Gly Gln Gly Leu Glu Trp Met
           35                  40                  45
   Gly Arg Ile Met Pro Leu Val Gly Leu Pro Glu Tyr Ala Glu Arg Phe
       50                  55                  60
   Gln Glu Arg Val Thr Phe Thr Ala Asp Lys Ser Thr Arg Thr Ala Tyr
   65                  70                  75                  80
   Met Asp Leu Thr Ser Leu Ser Ser Asp Asp Thr Ala Phe Tyr Phe Cys
                   85                  90                  95
   Ala Val Gly Val Met Lys Ile Phe Gly Glu Val Pro Leu Asn Leu Asp
               100                 105                 110
   Phe Trp Gly Gln Gly Thr Leu Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Lys Val Glu Pro
       210                 215                 220
   Lys Ser Cys Asp Lys Thr His Thr Cys Pro Pro Cys Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Asp Glu Leu Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
   420 425 430
   Ser Val Met His Glu Ala Pro His Asn His Tyr Thr Gln Lys Ser Leu
   435 440 445
   Ser Leu Ser Pro Gly Lys
   450
<210> 27
   <211> 215
   <212> PRT
   <213> Artificial
<220>
<210> 28
   <211> 456
   <212> PRT
   <213> Artificial
<220>
   <223> Amino acid sequence of entire heavy chain of e2d073 IgG antibody
<400> 28
   Gln Val Gln Leu Val Gln Ser Gly Ala Glu Val Lys Lys Pro Gly Ser
   1               5                   10                  15
   Ser Val Lys Val Ser Cys Lys Ala Ser Gly Ala Ser Phe Asn Ser Phe
               20                  25                  30
   Ala Ile Asp Trp Val Arg Gln Ala Pro Gly Gln Gly Leu Glu Trp Met
           35                  40                  45
   Gly Arg Ile Ile Pro Ile Ala Asp Val Ser Asp Tyr Ala Gln Lys Phe
       50                  55                  60
   Gln Gly Arg Val Thr Ile Thr Ala Asp Lys Ser Thr Arg Thr Ala Tyr
   65                  70                  75                  80
   Met Glu Leu Thr Thr Leu Arg Ser Asp Asp Thr Ala Val Tyr Tyr Cys
                   85                  90                  95
   Ala Thr Ser Pro Met Leu Thr Phe Gly Gly Pro Asn Ala Phe Gly Ala
               100                 105                 110
   Phe Asp Val Trp Gly Gln Gly Thr Met Val Thr Val Ser Ser Ala Ser
           115                 120                 125
   Thr Lys Gly Pro Ser Val Phe Pro Leu Ala Pro Ser Ser Lys Ser Thr
       130                 135                 140
   Ser Gly Gly Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Ser Leu Gly Thr Gln Thr Tyr Ile
           195                 200                 205
   Cys Asn Val Asn His Lys Pro Ser Asn Thr Lys Val Asp Lys Lys Val
       210                 215                 220
   Glu Pro Lys Ser Cys Asp Lys Thr His Thr Cys Pro Pro Cys Pro Ala
   225                 230                 235                 240
   Pro Glu Leu Leu Gly Gly Pro Ser Val Phe Leu Phe Pro Pro Lys Pro
                   245                 250                 255
   Lys Asp Thr Leu Met Ile Ser Arg Thr Pro Glu Val Thr Cys Val Val
               260                 265                 270
   Val Asp Val Ser His Glu Asp Pro Glu Val Lys Phe Asn Trp Tyr Val
           275                 280                 285
   Asp Gly Val Glu Val His Asn Ala Lys Thr Lys Pro Arg Glu Glu Gln
       290                 295                 300
   Tyr Asn Ser Thr Tyr Arg Val Val Ser Val Leu Thr Val Leu His Gln
   305                 310                 315                 320
   Asp Trp Leu Asn Gly Lys Glu Tyr Lys Cys Lys Val Ser Asn Lys Ala
                   325                 330                 335
   Leu Pro Ala Pro Ile Glu Lys Thr Ile Ser Lys Ala Lys Gly Gln Pro
               340                 345                 350
   Arg Glu Pro Gln Val Tyr Thr Leu Pro Pro Ser Arg Asp Glu Leu Thr
           355                 360                 365
   Lys Asn Gln Val Ser Leu Thr Cys Leu Val Lys Gly Phe Tyr Pro Ser
       370                 375                 380
   Asp Ile Ala Val Glu Trp Glu Ser Asn Gly Gln Pro Glu Asn Asn Tyr
   385                 390                 395                 400
   Lys Thr Thr Pro Pro Val Leu Asp Ser Asp Gly Ser Phe Phe Leu Tyr
                   405                 410                 415
   Ser Lys Leu Thr Val Asp Lys Ser Arg Trp Gln Gln Gly Asn Val Phe
   420 425 430
   Ser Cys Ser Val Met His Glu Ala Pro His Asn His Tyr Thr Gln Lys
   435 440 445
   Ser Leu Ser Leu Ser Pro Gly Lys
   450 455
<210> 29
   <211> 215
   <212> PRT
   <213> Artificial
<220>
<210> 30
   <211> 454
   <212> PRT
   <213> Artificial
<220>
   <223> Amino acid sequence of entire heavy chain of e2d081 IgG antibody
<400> 30
   Gln Val Gln Leu Val Gln Ser Gly Ser Asp Val Lys Lys Pro Gly Ser
   1 5 10 15
   Ser Val Arg Val Ser Cys Lys Ala Ser Gly Gly Ser Phe Asn Ser Tyr
   20 25 30
   Ala Val Asn Trp Val Arg Gln Ala Pro Gly Gln Gly Leu Glu Trp Met
   35 40 45
   Gly Arg Ile Met Pro Leu Val Gly Leu Pro Glu Tyr Ala Glu Arg Phe
   50 55 60
   Gln Glu Arg Val Thr Phe Thr Ala Asp Lys Ser Thr Arg Thr Ala Tyr
   65 70 75 80
   Met Asp Leu Thr Ser Leu Ser Ser Asp Asp Thr Ala Phe Tyr Phe Cys
   85 90 95
   Ala Val Gly Val Met Lys Ile Phe Gly Glu Val Pro Leu Asn Leu Asp
   100 105 110
   Phe Trp Gly Gln Gly Thr Leu Val Thr Val Ser Ser Ala Ser Thr Lys
   115 120 125
   Gly Pro Ser Val Phe Pro Leu Ala Pro Ser Ser Lys Ser Thr Ser Gly
   130 135 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Lys Val Glu Pro
       210                 215                 220
   Lys Ser Cys Asp Lys Thr His Thr Cys Pro Pro Cys Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Asp Glu Leu Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
               420                 425                 430
   Ser Val Met His Glu Ala Pro His Asn His Tyr Thr Gln Lys Ser Leu
           435                 440                 445
   Ser Leu Ser Pro Gly Lys
       450
<210> 31
   <211> 215
   <212> PRT
   <213> Artificial
<220>
   <223> Amino acid sequence of entire light chain of e2d081 IgG antibody
<400> 31
   Gln Ser Val Leu Thr Gln Pro Pro Ser Val Ser Ala Ala Pro Gly Gln
   1               5                   10                  15
   Lys Val Thr Ile Ser Cys Ser Gly Thr Ser Ser Asn Ile Gly Asp Asn
               20                  25                  30
   Tyr Val Ser Trp Tyr Gln His Leu Pro Gly Ser Ala Pro Lys Leu Leu
           35                  40                  45
   Ile Tyr Asp Asn Asn Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Phe Tyr Cys Gly Thr Trp Asp Ser Ser Leu
                   85                  90                  95
   Ser Ser Val Val Phe Gly Gly Gly Thr Lys Leu Thr Val Leu Gly Gln
               100                 105                 110
   Pro Lys Ala Ala Pro Ser Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Ser Ser Pro Val Lys
   145                 150                 155                 160
   Ala Gly Val Glu Thr Thr Thr Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Lys Ser Tyr Ser Cys Gln Val Thr His Glu Gly Ser Thr Val Glu Lys
           195                 200                 205
   Thr Val Ala Pro Thr Glu Cys
       210                 215
<210> 32
   <211> 488
   <212> PRT
   <213> Artificial
<220>
   <223> Amino acid sequence of whole e2d066 scFv antibody
<400> 32
   Gln Val Gln Leu Val Gln Ser Gly Ser Asp Val Lys Lys Pro Gly Ser
   1               5                   10                  15
   Ser Val Arg Val Ser Cys Lys Ala Ser Gly Gly Ser Phe Asn Ser Tyr
               20                  25                  30
   Ala Val Asn Trp Val Arg Gln Ala Pro Gly Gln Gly Leu Glu Trp Met
           35                  40                  45
   Gly Arg Ile Met Pro Leu Val Gly Leu Pro Glu Tyr Ala Glu Arg Phe
       50                  55                  60
   Gln Glu Arg Val Thr Phe Thr Ala Asp Lys Ser Thr Arg Thr Ala Tyr
   65                  70                  75                  80
   Met Asp Leu Thr Ser Leu Ser Ser Asp Asp Thr Ala Phe Tyr Phe Cys
                   85                  90                  95
   Ala Val Gly Val Met Lys Ile Phe Gly Glu Val Pro Leu Asn Leu Asp
               100                 105                 110
   Phe Trp Gly Gln Gly Thr Leu Val Thr Val Ser Ser Gly Gly Gly Gly
           115                 120                 125
   Ser Gly Gly Gly Gly Ser Gly Gly Gly Gly Ser Met Ala Gln Ala Val
       130                 135                 140
   Leu Thr Gln Pro Ser Ser Val Ser Gly Ser Pro Gly Gln Ser Ile Thr
   145                 150                 155                 160
   Ile Ser Cys Thr Gly Thr Ser Asn Asp Val Gly Ser Tyr Arg Tyr Val
                   165                 170                 175
   Ser Trp Phe Gln Gln His Pro Gly Lys Ala Pro Lys Leu Met Ile Tyr
               180                 185                 190
   Asp Val Asn Lys Arg Pro Ser Gly Val Ser Ser Arg Phe Ser Gly Ser
           195                 200                 205
   Lys Ser Gly Asn Thr Ala Ser Leu Thr Ile Ser Gly Leu Gln Ala Glu
       210                 215                 220
   Asp Glu Ala Asn Tyr Tyr Cys Ser Ser Tyr Thr Arg Ser Ser Ser Leu
   225                 230                 235                 240
   Ala Phe Gly Gly Gly Thr Lys Leu Thr Val Leu Ser Gln Pro Lys Ala
                   245                 250                 255
   Ala Pro Ser Val Thr Leu Phe Pro Pro Ser Ser Glu Glu Leu Gln Ala
               260                 265                 270
   Asn Lys Ala Thr Leu Val Cys Leu Ile Ser Asp Phe Tyr Pro Gly Ala
           275                 280                 285
   Val Thr Val Ala Trp Lys Ala Asp Ser Ser Pro Val Lys Ala Gly Val
       290                 295                 300
   Glu Thr Thr Thr Pro Ser Lys Gln Ser Asn Asn Lys Tyr Ala Ala Ser
   305                 310                 315                 320
   Ser Tyr Leu Ser Leu Thr Pro Glu Gln Trp Lys Ser His Lys Ser Tyr
                   325                 330                 335
   Ser Cys Gln Val Thr His Glu Gly Ser Thr Val Glu Lys Thr Val Ala
               340                 345                 350
   Pro Thr Glu Cys Ser Ala Arg Gln Ser Thr Ala Gln His Asp Glu Ala
           355                 360                 365
   Val Asp Asn Lys Phe Asn Lys Glu Gln Gln Asn Ala Phe Tyr Glu Ile
       370                 375                 380
   Leu His Leu Pro Asn Leu Asn Glu Glu Gln Arg Asn Ala Phe Ile Gln
   385                 390                 395                 400
   Ser Leu Lys Asp Asp Pro Ser Gln Ser Ala Asn Leu Leu Ala Glu Ala
                   405                 410                 415
   Lys Lys Leu Asn Asp Ala Gln Ala Pro Lys Val Asp Asn Lys Phe Asn
               420                 425                 430
   Lys Glu Gln Gln Asn Ala Phe Tyr Glu Ile Leu His Leu Pro Asn Leu
           435                 440                 445
   Asn Glu Glu Gln Arg Asn Ala Phe Ile Gln Ser Leu Lys Asp Asp Pro
       450                 455                 460
   Ser Gln Ser Ala Asn Leu Leu Ala Glu Ala Lys Lys Leu Asn Asp Ala
   465                 470                 475                 480
   Gln Ala Pro Lys Val Asp Ala Asn
                   485
<210> 33
   <211> 490
   <212> PRT
   <213> Artificial
<220>
   <223> Amino acid sequence of whole e2d073 scFv antibody
<400> 33
   Gln Val Gln Leu Val Gln Ser Gly Ala Glu Val Lys Lys Pro Gly Ser
   1               5                   10                  15
   Ser Val Lys Val Ser Cys Lys Ala Ser Gly Ala Ser Phe Asn Ser Phe
               20                  25                  30
   Ala Ile Asp Trp Val Arg Gln Ala Pro Gly Gln Gly Leu Glu Trp Met
           35                  40                  45
   Gly Arg Ile Ile Pro Ile Ala Asp Val Ser Asp Tyr Ala Gln Lys Phe
       50                  55                  60
   Gln Gly Arg Val Thr Ile Thr Ala Asp Lys Ser Thr Arg Thr Ala Tyr
   65                  70                  75                  80
   Met Glu Leu Thr Thr Leu Arg Ser Asp Asp Thr Ala Val Tyr Tyr Cys
                   85                  90                  95
   Ala Thr Ser Pro Met Leu Thr Phe Gly Gly Pro Asn Ala Phe Gly Ala
               100                 105                 110
   Phe Asp Val Trp Gly Gln Gly Thr Met Val Thr Val Ser Ser Gly Gly
           115                 120                 125
   Gly Gly Ser Gly Gly Gly Gly Ser Gly Gly Gly Gly Ser Met Ala Asp
       130                 135                 140
   Ile Gln Met Thr Gln Ser Pro Ser Ser Leu Ser Ala Ser Val Gly Asp
   145                 150                 155                 160
   Arg Val Thr Ile Thr Cys Arg Ala Ser Gln Ser Ile Ser Ser Tyr Leu
                   165                 170                 175
   Asn Trp Tyr Gln Gln Lys Pro Gly Lys Ala Pro Lys Leu Leu Ile Tyr
               180                 185                 190
   Ala Ala Ser Ser Leu Gln Ser Gly Val Pro Ser Arg Phe Ser Gly Ser
           195                 200                 205
   Gly Ser Gly Thr Asp Phe Thr Leu Thr Val Ser Ser Leu Gln Pro Glu
       210                 215                 220
   Asp Phe Ala Thr Tyr Tyr Cys Gln Gln Ser Tyr Ser Thr Pro Gln Phe
   225                 230                 235                 240
   Thr Phe Gly Pro Gly Thr Lys Val Asp Ile Lys Arg Thr Val Ala Ala
                   245                 250                 255
   Pro Ser Val Phe Ile Phe Pro Pro Ser Asp Glu Gln Leu Lys Ser Gly
               260                 265                 270
   Thr Ala Ser Val Val Cys Leu Leu Asn Asn Phe Tyr Pro Arg Glu Ala
           275                 280                 285
   Lys Val Gln Trp Lys Val Asp Asn Ala Leu Gln Ser Gly Asn Ser Gln
       290                 295                 300
   Glu Ser Val Thr Glu Gln Asp Ser Lys Asp Ser Thr Tyr Ser Leu Ser
   305                 310                 315                 320
   Ser Thr Leu Thr Leu Ser Lys Ala Asp Tyr Glu Lys His Lys Val Tyr
                   325                 330                 335
   Ala Cys Glu Val Thr His Gln Gly Leu Ser Ser Pro Val Thr Lys Ser
               340                 345                 350
   Phe Asn Arg Gly Glu Cys Ser Ala Arg Gln Ser Thr Ala Gln His Asp
   355 360 365
   Glu Ala Val Asp Asn Lys Phe Asn Lys Glu Gln Gln Asn Ala Phe Tyr
   370 375 380
   Glu Ile Leu His Leu Pro Asn Leu Asn Glu Glu Gln Arg Asn Ala Phe
   385 390 395 400
   Ile Gln Ser Leu Lys Asp Asp Pro Ser Gln Ser Ala Asn Leu Leu Ala
   405 410 415
   Glu Ala Lys Lys Leu Asn Asp Ala Gln Ala Pro Lys Val Asp Asn Lys
   420 425 430
   Phe Asn Lys Glu Gln Gln Asn Ala Phe Tyr Glu Ile Leu His Leu Pro
   435 440 445
   Asn Leu Asn Glu Glu Gln Arg Asn Ala Phe Ile Gln Ser Leu Lys Asp
   450 455 460
   Asp Pro Ser Gln Ser Ala Asn Leu Leu Ala Glu Ala Lys Lys Leu Asn
   465 470 475 480
   Asp Ala Gln Ala Pro Lys Val Asp Ala Asn
   485 490
<210> 34
   <211> 488
   <212> PRT
   <213> Artificial
<220>
   <223> Amino acid sequence of whole e2d081 scFv antibody
<400> 34
   Gln Val Gln Leu Val Gln Ser Gly Ser Asp Val Lys Lys Pro Gly Ser
   1               5                   10                  15
   Ser Val Arg Val Ser Cys Lys Ala Ser Gly Gly Ser Phe Asn Ser Tyr
               20                  25                  30
   Ala Val Asn Trp Val Arg Gln Ala Pro Gly Gln Gly Leu Glu Trp Met
           35                  40                  45
   Gly Arg Ile Met Pro Leu Val Gly Leu Pro Glu Tyr Ala Glu Arg Phe
       50                  55                  60
   Gln Glu Arg Val Thr Phe Thr Ala Asp Lys Ser Thr Arg Thr Ala Tyr
   65                  70                  75                  80
   Met Asp Leu Thr Ser Leu Ser Ser Asp Asp Thr Ala Phe Tyr Phe Cys
                   85                  90                  95
   Ala Val Gly Val Met Lys Ile Phe Gly Glu Val Pro Leu Asn Leu Asp
               100                 105                 110
   Phe Trp Gly Gln Gly Thr Leu Val Thr Val Ser Ser Gly Gly Gly Gly
           115                 120                 125
   Ser Gly Gly Gly Gly Ser Gly Gly Gly Gly Ser Met Ala Gln Ser Val
       130                 135                 140
   Leu Thr Gln Pro Pro Ser Val Ser Ala Ala Pro Gly Gln Lys Val Thr
   145                 150                 155                 160
   Ile Ser Cys Ser Gly Thr Ser Ser Asn Ile Gly Asp Asn Tyr Val Ser
                   165                 170                 175
   Trp Tyr Gln His Leu Pro Gly Ser Ala Pro Lys Leu Leu Ile Tyr Asp
               180                 185                 190
   Asn Asn Lys Arg Pro Ser Gly Ile Pro Asp Arg Phe Ser Gly Ser Lys
           195                 200                 205
   Ser Gly Thr Ser Ala Thr Leu Gly Ile Thr Gly Leu Gln Thr Gly Asp
       210                 215                 220
   Glu Ala Asp Phe Tyr Cys Gly Thr Trp Asp Ser Ser Leu Ser Ser Val
   225                 230                 235                 240
   Val Phe Gly Gly Gly Thr Lys Leu Thr Val Leu Gly Gln Pro Lys Ala
                   245                 250                 255
   Ala Pro Ser Val Thr Leu Phe Pro Pro Ser Ser Glu Glu Leu Gln Ala
               260                 265                 270
   Asn Lys Ala Thr Leu Val Cys Leu Ile Ser Asp Phe Tyr Pro Gly Ala
           275                 280                 285
   Val Thr Val Ala Trp Lys Ala Asp Ser Ser Pro Val Lys Ala Gly Val
       290                 295                 300
   Glu Thr Thr Thr Pro Ser Lys Gln Ser Asn Asn Lys Tyr Ala Ala Ser
   305                 310                 315                 320
   Ser Tyr Leu Ser Leu Thr Pro Glu Gln Trp Lys Ser His Lys Ser Tyr
                   325                 330                 335
   Ser Cys Gln Val Thr His Glu Gly Ser Thr Val Glu Lys Thr Val Ala
               340                 345                 350
   Pro Thr Glu Cys Ser Ala Arg Gln Ser Thr Ala Gln His Asp Glu Ala
           355                 360                 365
   Val Asp Asn Lys Phe Asn Lys Glu Gln Gln Asn Ala Phe Tyr Glu Ile
       370                 375                 380
   Leu His Leu Pro Asn Leu Asn Glu Glu Gln Arg Asn Ala Phe Ile Gln
   385                 390                 395                 400
   Ser Leu Lys Asp Asp Pro Ser Gln Ser Ala Asn Leu Leu Ala Glu Ala
                   405                 410                 415
   Lys Lys Leu Asn Asp Ala Gln Ala Pro Lys Val Asp Asn Lys Phe Asn
               420                 425                 430
   Lys Glu Gln Gln Asn Ala Phe Tyr Glu Ile Leu His Leu Pro Asn Leu
           435                 440                 445
   Asn Glu Glu Gln Arg Asn Ala Phe Ile Gln Ser Leu Lys Asp Asp Pro
       450                 455                 460
   Ser Gln Ser Ala Asn Leu Leu Ala Glu Ala Lys Lys Leu Asn Asp Ala
   465                 470                 475                 480
   Gln Ala Pro Lys Val Asp Ala Asn
                   485
<210> 35
   <211> 15
   <212> PRT
   <213> Artificial
<220>
   <223> Amino acid sequence of linker
<400> 35
   Gly Gly Gly Gly Ser Gly Gly Gly Gly Ser Gly Gly Gly Gly Ser
   1               5                   10                  15
<210> 36
   <211> 333
   <212> PRT
   <213> Artificial
<220>
   <223> Amino acid sequence of E2 protein of TH strain
<400> 36
   Ala Thr Tyr Val Thr Gly Gly Ser Glu Ala Arg Gly Ala Ser Gly Leu
   1               5                   10                  15
   Ala Asn Leu Phe Ser Phe Gly Ala Ser Gln Lys Ile Gln Leu Ile Asn
               20                  25                  30
   Thr Asn Gly Ser Trp His Ile Asn Arg Thr Ala Leu Asn Cys Asn Asp
           35                  40                  45
   Ser Leu His Thr Gly Phe Leu Ala Ala Leu Phe Tyr Thr His Lys Phe
       50                  55                  60
   Asn Ala Ser Gly Cys Pro Glu Arg Met Ala Ser Cys Arg Pro Ile Glu
   65                  70                  75                  80
   Glu Phe Ala Gln Gly Tyr Gly Pro Ile Thr Tyr Ala Glu Pro Ser Pro
                   85                  90                  95
   Ser Asp Gln Arg Pro Tyr Cys Trp His Tyr Ala Pro Arg Pro Cys Gly
               100                 105                 110
   Ile Ile Pro Ala Ser Gln Val Cys Gly Pro Val Tyr Cys Phe Thr Pro
           115                 120                 125
   Ser Pro Val Val Val Gly Thr Thr Asp Arg Ser Gly Ala Pro Thr Tyr
       130                 135                 140
   Asn Trp Gly Ala Asn Glu Thr Asp Val Leu Tyr Leu Asn Asn Thr Arg
   145                 150                 155                 160
   Pro Pro Gln Gly Asn Trp Phe Gly Cys Thr Trp Met Asn Gly Thr Gly
                   165                 170                 175
   Phe Thr Lys Thr Cys Gly Gly Pro Pro Cys Asn Ile Gly Gly Gly Gly
               180                 185                 190
   Asn Asn Asn Thr Leu Thr Cys Pro Thr Asp Cys Phe Arg Lys His Pro
           195                 200                 205
   Glu Ala Thr Tyr Thr Lys Cys Gly Ser Gly Pro Trp Leu Thr Pro Arg
       210                 215                 220
   Cys Met Val Asp Tyr Pro Tyr Arg Leu Trp His Tyr Pro Cys Thr Val
   225                 230                 235                 240
   Asn Phe Thr Ile Phe Lys Val Arg Met Tyr Val Gly Gly Val Glu His
   245 250 255
   Arg Leu Asn Ala Ala Cys Asn Trp Thr Arg Gly Glu Arg Cys Asn Leu
   260 265 270
   Glu Asp Arg Asp Arg Ser Glu Leu Ser Pro Leu Leu Leu Ser Thr Thr
   275 280 285
   Glu Trp Gln Val Leu Pro Cys Ser Phe Thr Thr Leu Pro Ala Leu Ser
   290 295 300
   Thr Gly Leu Ile His Leu His Gln Asn Ile Val Asp Val Gln Tyr Leu
   305 310 315 320
   Tyr Gly Ile Gly Ser Ala Val Val Ser Tyr Ala Ile Lys
   325 330
<210> 37
   <211> 12
   <212> PRT
   <213> Artificial
<220>
   <223> Amino acid sequence of TH E2 protein-derived peptide
<400> 37
   Gln Leu Ile Asn Thr Asn Gly Ser Trp His Ile Asn
   1 5 10
<210> 38
   <211> 105
   <212> PRT
   <213> Artificial
<220>
   <223> Amino acid sequence of e2d066 CL region
<400> 38
   Ser Gln Pro Lys Ala Ala Pro Ser Val Thr Leu Phe Pro Pro Ser Ser
   1 5 10 15
   Glu Glu Leu Gln Ala Asn Lys Ala Thr Leu Val Cys Leu Ile Ser Asp
   20 25 30
   Phe Tyr Pro Gly Ala Val Thr Val Ala Trp Lys Ala Asp Ser Ser Pro
   35 40 45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Lys Ser Tyr Ser Cys Gln Val Thr His Glu Gly Ser Thr Val
                   85                  90                  95
   Glu Lys Thr Val Ala Pro Thr Glu Cys
               100                 105
<210> 39
   <211> 107
   <212> PRT
   <213> Artificial
<220>
   <223> Amino acid sequence of e2d073 CL region
<400> 39
<210> 40
   <211> 105
   <212> PRT
   <213> Artificial
<220>
   <223> Amino acid sequence of e2d081 CL region
<400> 40
   Gly Gln Pro Lys Ala Ala Pro Ser Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Lys Ser Tyr Ser Cys Gln Val Thr His Glu Gly Ser Thr Val
                   85                  90                  95
   Glu Lys Thr Val Ala Pro Thr Glu Cys
               100                 105
<210> 41
   <211> 3030
   <212> PRT
   <213> Artificial
<220> <223> precursor protein of chimeric HCV particle of TH strain and JFH-1 strain (TH/JFH-1 HCVcc)
<400> 41
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Thr Gly Asp Met Ile Met His Thr Pro
       210                 215                 220
   Gly Cys Val Pro Cys Val Arg Glu Asn Asn Ser Ser Arg Cys Trp Ala
   225                 230                 235                 240
   Ala Leu Thr Pro Thr Leu Ala Ala Arg Asn Ala Ser Val Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Arg His Glu Thr Val Gln Asp Cys
       290                 295                 300
   Asn Cys Ser Ile Tyr Pro Gly His Val Ser Gly His Arg Met Ala Trp
   305                 310                 315                 320
   Asp Met Met Met Asn Trp Ser Pro Thr Thr Ala Leu Leu Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Ala Gly Asn Trp
           355                 360                 365
   Ala Lys Val Leu Ile Val Leu Leu Leu Phe Ala Gly Val Asp Gly Ala
       370                 375                 380
   Thr Tyr Val Thr Gly Gly Ser Glu Ala Arg Gly Ala Ser Gly Leu Ala
   385                 390                 395                 400
   Asn Leu Phe Ser Phe Gly Ala Ser Gln Lys Ile Gln Leu Ile Asn Thr
                   405                 410                 415
   Asn Gly Ser Trp His Ile Asn Arg Thr Ala Leu Asn Cys Asn Asp Ser
               420                 425                 430
   Leu His Thr Gly Phe Leu Ala Ala Leu Phe Tyr Thr His Lys Phe Asn
           435                 440                 445
   Ala Ser Gly Cys Pro Glu Arg Met Ala Ser Cys Arg Pro Ile Glu Glu
       450                 455                 460
   Phe Ala Gln Gly Tyr Gly Pro Ile Thr Tyr Ala Glu Pro Ser Pro Ser
   465                 470                 475                 480
   Asp Gln Arg Pro Tyr Cys Trp His Tyr Ala Pro Arg Pro Cys Gly Ile
                   485                 490                 495
   Ile Pro Ala Ser Gln Val Cys Gly Pro Val Tyr Cys Phe Thr Pro Ser
               500                 505                 510
   Pro Val Val Val Gly Thr Thr Asp Arg Ser Gly Ala Pro Thr Tyr Asn
           515                 520                 525
   Trp Gly Ala Asn Glu Thr Asp Val Leu Tyr Leu Asn Asn Thr Arg Pro
       530                 535                 540
   Pro Gln Gly Asn Trp Phe Gly Cys Thr Trp Met Asn Gly Thr Gly Phe
   545                 550                 555                 560
   Thr Lys Thr Cys Gly Gly Pro Pro Cys Asn Ile Gly Gly Gly Gly Asn
                   565                 570                 575
   Asn Asn Thr Leu Thr Cys Pro Thr Asp Cys Phe Arg Lys His Pro Glu
               580                 585                 590
   Ala Thr Tyr Thr Lys Cys Gly Ser Gly Pro Trp Leu Thr Pro Arg Cys
           595                 600                 605
   Met Val Asp Tyr Pro Tyr Arg Leu Trp His Tyr Pro Cys Thr Val Asn
       610                 615                 620
   Phe Thr Ile Phe Lys Val Arg Met Tyr Val Gly Gly Val Glu His Arg
   625                 630                 635                 640
   Leu Asn Ala Ala Cys Asn Trp Thr Arg Gly Glu Arg Cys Asn Leu Glu
                   645                 650                 655
   Asp Arg Asp Arg Ser Glu Leu Ser Pro Leu Leu Leu Ser Thr Thr Glu
               660                 665                 670
   Trp Gln Val Leu Pro Cys Ser Phe Thr Thr Leu Pro Ala Leu Ser Thr
           675                 680                 685
   Gly Leu Ile His Leu His Gln Asn Ile Val Asp Val Gln Tyr Leu Tyr
       690                 695                 700
   Gly Ile Gly Ser Ala Val Val Ser Tyr Ala Ile Lys Trp Glu Tyr Val
   705                 710                 715                 720
   Leu Leu Leu Phe Leu Leu Leu Ala Asp Ala Arg Val Cys Ala Cys Leu
                   725                 730                 735
   Trp Met Met Leu Leu Ile Ala Gln Ala Glu Ala Ala Leu Glu Asn Leu
               740                 745                 750
   Val Val Leu Asn Ala Ala Ser Leu Ala Gly Ala His Gly Leu Leu Ser
           755                 760                 765
   Phe Leu Val Phe Phe Cys Ala Ala Trp Tyr Ile Lys Gly Arg Leu Ile
       770                 775                 780
   Pro Gly Ala Ala Tyr Ala Phe Tyr Gly Val Trp Pro Leu Leu Leu Leu
   785                 790                 795                 800
   Leu Leu Ala Leu Pro Pro Arg Ala Tyr Ala Met Asp Arg Glu Met Ala
                   805                 810                 815
   Ala Ser Cys Gly Gly Ala Val Phe Val Gly Leu Ala Phe Leu Thr Leu
               820                 825                 830
   Ser Pro His Tyr Lys Ala Phe Leu Ala Lys Leu Leu Trp Trp Leu Cys
           835                 840                 845
   Tyr Leu Leu Thr Leu Gly Glu Ala Met Ile Gln Glu Trp Val Pro Pro
       850                 855                 860
   Met Gln Val Arg Gly Gly Arg Asp Gly Ile Ala Trp Ala Val Thr Ile
   865                 870                 875                 880
   Phe Cys Pro Gly Val Val Phe Asp Ile Thr Lys Trp Leu Leu Ala Leu
                   885                 890                 895
   Leu Gly Pro Ala Tyr Leu Leu Arg Ala Ala Leu Thr His Val Pro Tyr
               900                 905                 910
   Phe Val Arg Ala His Ala Leu Ile Arg Val Cys Ala Leu Val Lys Gln
           915                 920                 925
   Leu Ala Gly Gly Arg Tyr Val Gln Val Ala Leu Leu Ala Leu Gly Arg
       930                 935                 940
   Trp Thr Gly Thr Tyr Ile Tyr Asp His Leu Thr Pro Met Ser Asp Trp
   945                 950                 955                 960
   Ala Ala Ser Gly Leu Arg Asp Leu Ala Val Ala Val Glu Pro Ile Ile
                   965                 970                 975
   Phe Ser Pro Met Glu Lys Lys Val Ile Val Trp Gly Ala Glu Thr Ala
               980                 985                 990
   Ala Cys Gly Asp Ile Leu His Gly  Leu Pro Val Ser Ala  Arg Leu Gly
           995                 1000                1005
   Gln Glu  Ile Leu Leu Gly Pro  Ala Asp Gly Tyr Thr  Ser Lys Gly
       1010                 1015                 1020
   Trp Lys  Leu Leu Ala Pro Ile  Thr Ala Tyr Ala Gln  Gln Thr Arg
       1025                 1030                 1035
   Gly Leu  Leu Gly Ala Ile Val  Val Ser Met Thr Gly  Arg Asp Arg
       1040                 1045                 1050
   Thr Glu  Gln Ala Gly Glu Val  Gln Ile Leu Ser Thr  Val Ser Gln
       1055                 1060                 1065
   Ser Phe  Leu Gly Thr Thr Ile  Ser Gly Val Leu Trp  Thr Val Tyr
       1070                 1075                 1080
   His Gly  Ala Gly Asn Lys Thr  Leu Ala Gly Leu Arg  Gly Pro Val
       1085                 1090                 1095
   Thr Gln  Met Tyr Ser Ser Ala  Glu Gly Asp Leu Val  Gly Trp Pro
       1100                 1105                 1110
   Ser Pro  Pro Gly Thr Lys Ser  Leu Glu Pro Cys Lys  Cys Gly Ala
       1115                 1120                 1125
   Val Asp  Leu Tyr Leu Val Thr  Arg Asn Ala Asp Val  Ile Pro Ala
       1130                 1135                 1140
   Arg Arg  Arg Gly Asp Lys Arg  Gly Ala Leu Leu Ser  Pro Arg Pro
       1145                 1150                 1155
   Ile Ser  Thr Leu Lys Gly Ser  Ser Gly Gly Pro Val  Leu Cys Pro
       1160                 1165                 1170
   Arg Gly  His Val Val Gly Leu  Phe Arg Ala Ala Val  Cys Ser Arg
       1175                 1180                 1185
   Gly Val  Ala Lys Ser Ile Asp  Phe Ile Pro Val Glu  Thr Leu Asp
       1190                 1195                 1200
   Val Val  Thr Arg Ser Pro Thr  Phe Ser Asp Asn Ser  Thr Pro Pro
       1205                 1210                 1215
   Ala Val  Pro Gln Thr Tyr Gln  Val Gly Tyr Leu His  Ala Pro Thr
       1220                 1225                 1230
   Gly Ser  Gly Lys Ser Thr Lys  Val Pro Val Ala Tyr  Ala Ala Gln
       1235                 1240                 1245
   Gly Tyr  Lys Val Leu Val Leu  Asn Pro Ser Val Ala  Ala Thr Leu
       1250                 1255                 1260
   Gly Phe  Gly Ala Tyr Leu Ser  Lys Ala His Gly Ile  Asn Pro Asn
       1265                 1270                 1275
   Ile Arg  Thr Gly Val Arg Thr  Val Met Thr Gly Glu  Ala Ile Thr
       1280                 1285                 1290
   Tyr Ser  Thr Tyr Gly Lys Phe  Leu Ala Asp Gly Gly  Cys Ala Ser
       1295                 1300                 1305
   Gly Ala  Tyr Asp Ile Ile Ile  Cys Asp Glu Cys His  Ala Val Asp
       1310                 1315                 1320
   Ala Thr  Ser Ile Leu Gly Ile  Gly Thr Val Leu Asp  Gln Ala Glu
       1325                 1330                 1335
   Thr Ala Gly Val Arg Leu Thr Val Leu Ala Thr Ala Thr Pro Pro
   1340 1345 1350
   Gly Ser Val Thr Thr Pro His Pro Asp Ile Glu Glu Val Gly Leu
   1355 1360 1365
   Gly Arg Glu Gly Glu Ile Pro Phe Tyr Gly Arg Ala Ile Pro Leu
   1370 1375 1380
   Ser Cys Ile Lys Gly Gly Arg His Leu Ile Phe Cys His Ser Lys
   1385 1390 1395
   Lys Lys Cys Asp Glu Leu Ala Ala Ala Leu Arg Gly Met Gly Leu
   1400 1405 1410
   Asn Ala Val Ala Tyr Tyr Arg Gly Leu Asp Val Ser Ile Ile Pro
   1415 1420 1425
   Ala Gln Gly Asp Val Val Val Val Ala Thr Asp Ala Leu Met Thr
   1430 1435 1440
   Gly Tyr Thr Gly Asp Phe Asp Ser Val Ile Asp Cys Asn Val Ala
   1445 1450 1455
   Val Thr Gln Ala Val Asp Phe Ser Leu Asp Pro Thr Phe Thr Ile
   1460 1465 1470
   Thr Thr Gln Thr Val Pro Gln Asp Ala Val Ser Arg Ser Gln Arg
   1475 1480 1485
   Arg Gly Arg Thr Gly Arg Gly Arg Gln Gly Thr Tyr Arg Tyr Val
   1490 1495 1500
   Ser Thr Gly Glu Arg Ala Ser Gly Met Phe Asp Ser Val Val Leu
   1505 1510 1515
   Cys Glu Cys Tyr Asp Ala Gly Ala Ala Trp Tyr Asp Leu Thr Pro
   1520 1525 1530
   Ala Glu Thr Thr Val Arg Leu Arg Ala Tyr Phe Asn Thr Pro Gly
   1535 1540 1545
   Leu Pro Val Cys Gln Asp His Leu Glu Phe Trp Glu Ala Val Phe
   1550 1555 1560
   Thr Gly Leu Thr His Ile Asp Ala His Phe Leu Ser Gln Thr Lys
   1565 1570 1575
   Gln Ala Gly Glu Asn Phe Ala Tyr Leu Val Ala Tyr Gln Ala Thr
   1580 1585 1590
   Val Cys Ala Arg Ala Lys Ala Pro Pro Pro Ser Trp Asp Ala Met
   1595 1600 1605
   Trp Lys Cys Leu Ala Arg Leu Lys Pro Thr Leu Ala Gly Pro Thr
   1610 1615 1620
   Pro Leu Leu Tyr Arg Leu Gly Pro Ile Thr Asn Glu Val Thr Leu
   1625 1630 1635
   Thr His Pro Gly Thr Lys Tyr Ile Ala Thr Cys Met Gln Ala Asp
   1640 1645 1650
   Leu Glu Val Met Thr Ser Thr Trp Val Leu Ala Gly Gly Val Leu
   1655 1660 1665
   Ala Ala Val Ala Ala Tyr Cys Leu Ala Thr Gly Cys Val Ser Ile
   1670 1675 1680
   Ile Gly Arg Leu His Val Asn Gln Arg Val Val Val Ala Pro Asp
   1685 1690 1695
   Lys Glu Val Leu Tyr Glu Ala Phe Asp Glu Met Glu Glu Cys Ala
   1700 1705 1710
   Ser Arg Ala Ala Leu Ile Glu Glu Gly Gln Arg Ile Ala Glu Met
   1715 1720 1725
   Leu Lys Ser Lys Ile Gln Gly Leu Leu Gln Gln Ala Ser Lys Gln
   1730 1735 1740
   Ala Gln Asp Ile Gln Pro Ala Met Gln Ala Ser Trp Pro Lys Val
   1745 1750 1755
   Glu Gln Phe Trp Ala Arg His Met Trp Asn Phe Ile Ser Gly Ile
   1760 1765 1770
   Gln Tyr Leu Ala Gly Leu Ser Thr Leu Pro Gly Asn Pro Ala Val
   1775 1780 1785
   Ala Ser Met Met Ala Phe Ser Ala Ala Leu Thr Ser Pro Leu Ser
   1790 1795 1800
   Thr Ser Thr Thr Ile Leu Leu Asn Ile Met Gly Gly Trp Leu Ala
   1805 1810 1815
   Ser Gln Ile Ala Pro Pro Ala Gly Ala Thr Gly Phe Val Val Ser
   1820 1825 1830
   Gly Leu Val Gly Ala Ala Val Gly Ser Ile Gly Leu Gly Lys Val
   1835 1840 1845
   Leu Val Asp Ile Leu Ala Gly Tyr Gly Ala Gly Ile Ser Gly Ala
   1850 1855 1860
   Leu Val Ala Phe Lys Ile Met Ser Gly Glu Lys Pro Ser Met Glu
   1865 1870 1875
   Asp Val Ile Asn Leu Leu Pro Gly Ile Leu Ser Pro Gly Ala Leu
   1880 1885 1890
   Val Val Gly Val Ile Cys Ala Ala Ile Leu Arg Arg His Val Gly
   1895 1900 1905
   Pro Gly Glu Gly Ala Val Gln Trp Met Asn Arg Leu Ile Ala Phe
   1910 1915 1920
   Ala Ser Arg Gly Asn His Val Ala Pro Thr His Tyr Val Thr Glu
   1925 1930 1935
   Ser Asp Ala Ser Gln Arg Val Thr Gln Leu Leu Gly Ser Leu Thr
   1940 1945 1950
   Ile Thr Ser Leu Leu Arg Arg Leu His Asn Trp Ile Thr Glu Asp
   1955 1960 1965
   Cys Pro Ile Pro Cys Ser Gly Ser Trp Leu Arg Asp Val Trp Asp
   1970 1975 1980
   Trp Val Cys Thr Ile Leu Thr Asp Phe Lys Asn Trp Leu Thr Ser
   1985 1990 1995
   Lys Leu Phe Pro Lys Leu Pro Gly Leu Pro Phe Ile Ser Cys Gln
   2000 2005 2010
   Lys Gly Tyr Lys Gly Val Trp Ala Gly Thr Gly Ile Met Thr Thr
   2015 2020 2025
   Arg Cys Pro Cys Gly Ala Asn Ile Ser Gly Asn Val Arg Leu Gly
   2030 2035 2040
   Ser Met  Arg Ile Thr Gly Pro  Lys Thr Cys Met Asn  Thr Trp Gln
       2045                 2050                 2055
   Gly Thr  Phe Pro Ile Asn Cys  Tyr Thr Glu Gly Gln  Cys Ala Pro
       2060                 2065                 2070
   Lys Pro  Pro Thr Asn Tyr Lys  Thr Ala Ile Trp Arg  Val Ala Ala
       2075                 2080                 2085
   Ser Glu  Tyr Ala Glu Val Thr  Gln His Gly Ser Tyr  Ser Tyr Val
       2090                 2095                 2100
   Thr Gly  Leu Thr Thr Asp Asn  Leu Lys Ile Pro Cys  Gln Leu Pro
       2105                 2110                 2115
   Ser Pro  Glu Phe Phe Ser Trp  Val Asp Gly Val Gln  Ile His Arg
       2120                 2125                 2130
   Phe Ala  Pro Thr Pro Lys Pro  Phe Phe Arg Asp Glu  Val Ser Phe
       2135                 2140                 2145
   Cys Val  Gly Leu Asn Ser Tyr  Ala Val Gly Ser Gln  Leu Pro Cys
       2150                 2155                 2160
   Glu Pro  Glu Pro Asp Ala Asp  Val Leu Arg Ser Met  Leu Thr Asp
       2165                 2170                 2175
   Pro Pro  His Ile Thr Ala Glu  Thr Ala Ala Arg Arg  Leu Ala Arg
       2180                 2185                 2190
   Gly Ser  Pro Pro Ser Glu Ala  Ser Ser Ser Val Ser  Gln Leu Ser
       2195                 2200                 2205
   Ala Pro  Ser Leu Arg Ala Thr  Cys Thr Thr His Ser  Asn Thr Tyr
       2210                 2215                 2220
   Asp Val  Asp Met Val Asp Ala  Asn Leu Leu Met Glu  Gly Gly Val
       2225                 2230                 2235
   Ala Gln  Thr Glu Pro Glu Ser  Arg Val Pro Val Leu  Asp Phe Leu
       2240                 2245                 2250
   Glu Pro  Met Ala Glu Glu Glu  Ser Asp Leu Glu Pro  Ser Ile Pro
       2255                 2260                 2265
   Ser Glu  Cys Met Leu Pro Arg  Ser Gly Phe Pro Arg  Ala Leu Pro
       2270                 2275                 2280
   Ala Trp Ala Arg Pro Asp Tyr Asn Pro Pro Leu Val Glu Ser Trp
   2285 2290 2295
   Arg Arg Pro Asp Tyr Gln Pro Pro Thr Val Ala Gly Cys Ala Leu
   2300 2305 2310
   Pro Pro Pro Lys Lys Ala Pro Thr Pro Pro Pro Arg Arg Arg Arg
   2315 2320 2325
   Thr Val Gly Leu Ser Glu Ser Thr Ile Ser Glu Ala Leu Gln Gln
   2330 2335 2340
   Leu Ala Ile Lys Thr Phe Gly Gln Pro Pro Ser Ser Gly Asp Ala
   2345 2350 2355
   Gly Ser Ser Thr Gly Ala Gly Ala Ala Glu Ser Gly Gly Pro Thr
   2360 2365 2370
   Ser Pro Gly Glu Pro Ala Pro Ser Glu Thr Gly Ser Ala Ser Ser
   2375 2380 2385
   Met Pro Pro Leu Glu Gly Glu Pro Gly Asp Pro Asp Leu Glu Ser
   2390 2395 2400
   Asp Gln Val Glu Leu Gln Pro Pro Pro Gln Gly Gly Gly Val Ala
   2405 2410 2415
   Pro Gly Ser Gly Ser Gly Ser Trp Ser Thr Cys Ser Glu Glu Asp
   2420 2425 2430
   Asp Thr Thr Val Cys Cys Ser Met Ser Tyr Ser Trp Thr Gly Ala
   2435 2440 2445
   Leu Ile Thr Pro Cys Ser Pro Glu Glu Glu Lys Leu Pro Ile Asn
   2450 2455 2460
   Pro Leu Ser Asn Ser Leu Leu Arg Tyr His Asn Lys Val Tyr Cys
   2465 2470 2475
   Thr Thr Ser Lys Ser Ala Ser Gln Arg Ala Lys Lys Val Thr Phe
   2480 2485 2490
   Asp Arg Thr Gln Val Leu Asp Ala His Tyr Asp Ser Val Leu Lys
   2495 2500 2505
   Asp Ile Lys Leu Ala Ala Ser Lys Val Ser Ala Arg Leu Leu Thr
   2510 2515 2520
   Leu Glu Glu Ala Cys Gln Leu Thr Pro Pro His Ser Ala Arg Ser
   2525 2530 2535
   Lys Tyr Gly Phe Gly Ala Lys Glu Val Arg Ser Leu Ser Gly Arg
   2540 2545 2550
   Ala Val Asn His Ile Lys Ser Val Trp Lys Asp Leu Leu Glu Asp
   2555 2560 2565
   Pro Gln Thr Pro Ile Pro Thr Thr Ile Met Ala Lys Asn Glu Val
   2570 2575 2580
   Phe Cys Val Asp Pro Ala Lys Gly Gly Lys Lys Pro Ala Arg Leu
   2585 2590 2595
   Ile Val Tyr Pro Asp Leu Gly Val Arg Val Cys Glu Lys Met Ala
   2600 2605 2610
   Leu Tyr Asp Ile Thr Gln Lys Leu Pro Gln Ala Val Met Gly Ala
   2615 2620 2625
   Ser Tyr Gly Phe Gln Tyr Ser Pro Ala Gln Arg Val Glu Tyr Leu
   2630 2635 2640
   Leu Lys Ala Trp Ala Glu Lys Lys Asp Pro Met Gly Phe Ser Tyr
   2645 2650 2655
   Asp Thr Arg Cys Phe Asp Ser Thr Val Thr Glu Arg Asp Ile Arg
   2660 2665 2670
   Thr Glu Glu Ser Ile Tyr Gln Ala Cys Ser Leu Pro Glu Glu Ala
   2675 2680 2685
   Arg Thr Ala Ile His Ser Leu Thr Glu Arg Leu Tyr Val Gly Gly
   2690 2695 2700
   Pro Met Phe Asn Ser Lys Gly Gln Thr Cys Gly Tyr Arg Arg Cys
   2705 2710 2715
   Arg Ala Ser Gly Val Leu Thr Thr Ser Met Gly Asn Thr Ile Thr
   2720 2725 2730
   Cys Tyr Val Lys Ala Leu Ala Ala Cys Lys Ala Ala Gly Ile Val
   2735 2740 2745
   Ala Pro Thr Met Leu Val Cys Gly Asp Asp Leu Val Val Ile Ser
       2750                2755                2760
   Glu Ser Gln Gly Thr Glu Glu Asp Glu Arg Asn Leu Arg Ala Phe
       2765                2770                2775
   Thr Glu Ala Met Thr Arg Tyr Ser Ala Pro Pro Gly Asp Pro Pro
       2780                2785                2790
   Arg Pro Glu Tyr Asp Leu Glu Leu Ile Thr Ser Cys Ser Ser Asn
       2795                2800                2805
   Val Ser Val Ala Leu Gly Pro Arg Gly Arg Arg Arg Tyr Tyr Leu
       2810                2815                2820
   Thr Arg Asp Pro Thr Thr Pro Leu Ala Arg Ala Ala Trp Glu Thr
       2825                2830                2835
   Val Arg His Ser Pro Ile Asn Ser Trp Leu Gly Asn Ile Ile Gln
       2840                2845                2850
   Tyr Ala Pro Thr Ile Trp Val Arg Met Val Leu Met Thr His Phe
       2855                2860                2865
   Phe Ser Ile Leu Met Val Gln Asp Thr Leu Asp Gln Asn Leu Asn
       2870                2875                2880
   Phe Glu Met Tyr Gly Ser Val Tyr Ser Val Asn Pro Leu Asp Leu
       2885                2890                2895
   Pro Ala Ile Ile Glu Arg Leu His Gly Leu Asp Ala Phe Ser Met
       2900                2905                2910
   His Thr Tyr Ser His His Glu Leu Thr Arg Val Ala Ser Ala Leu
       2915                2920                2925
   Arg Lys Leu Gly Ala Pro Pro Leu Arg Val Trp Lys Ser Arg Ala
       2930                2935                2940
   Arg Ala Val Arg Ala Ser Leu Ile Ser Arg Gly Gly Lys Ala Ala
       2945                2950                2955
   Val Cys Gly Arg Tyr Leu Phe Asn Trp Ala Val Lys Thr Lys Leu
       2960                2965                2970
   Lys Leu Thr Pro Leu Pro Glu Ala Arg Leu Leu Asp Leu Ser Ser
       2975                2980                2985
   Trp Phe  Thr Val Gly Ala Gly  Gly Gly Asp Ile Phe  His Ser Val
       2990                 2995                 3000
   Ser Arg  Ala Arg Pro Arg Ser  Leu Leu Phe Gly Leu  Leu Leu Leu
       3005                 3010                 3015
   Phe Val  Gly Val Gly Leu Phe  Leu Leu Pro Ala Arg
       3020                 3025                 3030

## Description

La présente invention concerne un domaine polypeptidique responsable des interactions entre une enveloppe rétrovirale du groupe d'interférence de HERV-W et les récepteurs de la famille hASCT.

Les rétrovirus endogènes humains (HERVs) constituent 8% du génome humain et sont impliqués à la fois dans des pathologies et dans des phénomènes non pathologiques.
La famille des rétrovirus endogènes humains W (HERV-W) est dérivée d'un élément rétroviral infectieux intégré dans la lignée germinale il y a 25 à 40 millions d'années. La protéine d'enveloppe de HERV-W, appelée également syncytine, est une glycoprotéine fusogène impliquée dans la formation de la couche syncytiotrophoblastique du placenta. Elle est codée par le gène env du locus proviral ERVW1 et synthétisée sous la forme d'un précurseur gPr73 qui est spécifiquement clivé en deux protéines matures, une sous unité de surface gp50 (SU) et une sous unité transmembranaires gp24 (TM).
In vitro, la syncytine de la famille HERV-W induit une fusion cellule à cellule dépendante de son interaction avec un récepteur-transporteur d'acides aminés de la famille ASCT (h-ASCT2, hASCT1). Des études phylogéniques ont alors montré que la syncytine est apparentée à un groupe de rétrovirus, comprenant notamment le virus endogène du chat RD114, le virus endogène du singe BaEV, des rétrovirus simiens et des rétrovirus aviaires : virus de la réticuloendothéliose aviaire REV-A et virus de la nécrose de la rate SNV, ayant tous en commun le récepteur-transporteur d'acides aminés neutres sodium dépendant de type 2 ou hASCT2 (Rasko et al, 1999, Proc. Natl. Acad. Sci. USA Vol. 96, pp. 2129-2134; Tailor et al, 1999 JOURNAL OF VIROLOGY, VOL 73(5) May 1999, p. 4470-4474). Ainsi, l'infection de cellules par des virus de ce groupe de rétrovirus conduit à une réduction spécifique du transport des acides aminés (Rasko et al., 1999). L'infection d'une cellule par un de ces rétrovirus (ou l'expression dans la cellule de l'une de ces enveloppes) empêche, par interférence (interaction) vis à vis d'un récepteur de la famille ASCT, l'infection de cette même cellule par un autre de ces rétrovirus ou la fusion avec une autre cellule exprimant une autre enveloppe. Par interférence vis-à-vis d'un récepteur de la famille ASCT, l'infection d'une cellule par un de ces rétrovirus empêche l'infection par un autre de ces rétrovirus. Tous ces rétrovirus appartiennent au même groupe d'interférence du virus HERV-W.
Les mécanismes de liaisons entre l'enveloppe et le récepteur ASCT restent obscurs, et à ce jour aucun domaine de liaison à un récepteur ASCT n'a été identifié et défini que ce soit dans la SU de la protéine d'enveloppe de HERV-W ou dans les SU de rétrovirus du même groupe d'interférence. Cette thématique est pourtant essentielle puisque l'inhibition de l'interaction enveloppe / récepteur ASCT permettrait en outre d'empêcher l'entrée d'un rétrovirus dans la cellule, et donc de bloquer son cycle de réplication, de bloquer le phénomène d'interaction enveloppe / récepteur ASCT et/ou de fusion cellulaire pouvant être impliqué dans la formation de tumeurs, dans la prolifération de cellules métastasiques ou dans des phénomènes de résistance à des médicaments (voir à titre d'illustration la publication « Cell fusion : A hidden enemy ?, Cancer Cell : May 2003 Vol.3), de bloquer le phénomène d'interaction enveloppe / récepteur ASCT et/ou de fusion cellulaire pouvant intervenir dans des maladies du système nerveux voire d'inhiber la fusion cellule-cellule impliquée dans la différenciation trophoblastique (vaccination contraceptive). De plus, l'inhibition de l'interaction enveloppe / récepteur hASCT pourrait empêcher la propagation de tumeur en s'opposant à une immunosuppression locale pouvant découler de l'interaction enveloppe / récepteur hASCT. En effet, il a été montré d'une part que l'infection de cellules par des virus de ce groupe de rétrovirus (notamment ceux induisant des immunodéficiences) conduit à une réduction spécifique du transport des acides aminés (Rasko et al, PNAS, Vol. 96, pp 2129-2134 (1999)), et d'autre part il est proposé un lien direct entre l'altération du transport des acides aminés et l'immunosuppression (Espinosa A, Villarreal LP., T-Ag inhibits implantation by EC cell derived embryoid bodies. Virus Genes. 2000;20(3):195-200; JE, Battini JL, Gottschalk RJ, Mazo I, Miller AD., The RD114/simian type D retrovirus receptor is a neutral amino acid transporter. Proc Natl Acad Sci USA. 1999 Mar 2;96(5):2129-34.). Ainsi, concernant les maladies du système nerveux, il est connu que les récepteurs hASCT sont impliqués dans le transport spécifique d'acides aminés neutres et que les cellules neuronales, pour la transmission des informations, utilisent de manière prépondérante des neuromédiateurs de nature polypeptique. Ainsi, la liaison de la protéine Env-HERV-W à des récepteurs devant normalement transporter les acides aminés requis pour la synthèse des neuromédiateurs peut affecter la capacité des neurones à synthétiser les neuromédiateurs en réduisant l'entrée des agonistes physiologiques que sont les acides aminés via les récepteurs ASCT. Par ailleurs, si des neurones, dont les réseaux inter-cellulaires forment des connexions indispensables à la transmission des informations circulant dans le cerveau et la moelle épinière, forment des syncytia suite à une fusion de plusieurs neurones induite par la protéine Env-HERV-W, tous les réseaux de transmission des informations se trouvent perturbés etr connectés au même « paquet cellulaire » fusionné et de plus l'activité de production des neuromédiateurs de chaque cellule n'est plus indivisualisée, ni connectée aux voies de conduction amont et aval (dendrites et axones) qui lui sont propres.

D'une manière surprenante, les inventeurs ont identifié la région polypeptidique responsable des interactions entre l'enveloppe d'un virus appartenant au groupe d'interférence HERV-W et un récepteur hASCT.

Ils ont effectivement identifié un domaine peptidique nécessaire à l'interaction entre l'enveloppe d'un virus appartenant au groupe d'interférence de HERV-W et un récepteur hASCT, défini en ce qu'il commence par une extrémité N-terminale et se termine par une extrémité C-terminale, et en ce que :
▪ l'extrémité N-terminale est définie par un motif, constitué par les acides aminés (Z)_{α}-Proline-Cystéine-X-Cystéine
   dans lequel
   Z est un acide aminé quelconque
   α est un nombre entier compris entre 2 et 30
   X est un acide aminé quelconque, ledit motif étant choisi parmi SEQ ID N°1 à SEQ ID N°29 et SEQ ID NO : 44 à SEQ ID NO : 72
▪ l'extrémité C-terminale est définie par un motif, constitué par les acides aminés Serine-Acide Aspartique-Xₐ-X_{b}-X_{c}-X_{d}-Xₑ-Acide Aspartique-X_{f}-X_{g}-(Z)_{β} dans lequel
   Xₐ, X_{b}, X_{c}, X_{d}, Xₑ, X_{f}, X_{g}, sont des acides aminés quelconques
   Z est un acide aminé quelconque
   β est un nombre entier compris entre 15 et 25, préférentiellement 20
   ledit motif étant choisi parmi SEQ ID N°30 à SEQ ID N°40,
et en ce que ledit domaine peptidique comprend, entre l'extrémité N terminale et l'extrémité C terminale au moins un motif choisi parmi les motifs suivants :
un motif constitué par les acides aminés Cystéine-Tyrosine-X₂-X₃-X₄-X₅-X₆-Cystéine, dans lequel X₂, X₃, X₄, X₅, X₆ sont des acides aminés quelconques, ledit motif correspondant à la SEQ ID N°41
un motif constitué par les acides aminés Cystéine-X₇-X₈-X₉-X₁₀-X₁₁- X₁₂-X₁₃-X-₁₄-X₁₅-Cystéine-Trytophane, dans lequel X₇, X₈, X₉, X_{10,} X₁₁, X₁₂, X₁₃, X₁₄, X₁₅ sont des acides aminés quelconques, ledit motif correspondant à la SEQ ID N°42 ou SEQ ID NO : 73.
Ainsi l'invention concerne un domaine peptidique de l'enveloppe d'un virus appartenant au groupe d'interférence de HERV-W, capable d'interagir avec un récepteur hASCT, défini en ce qu'il commence par une extrémité N terminale et se termine par une extrémité C terminale, et en ce que :
- l'extrémité N-terminale est définie par un motif choisi parmi SEQ ID N°1 à SEQ ID N°29,
- l'extrémité C terminale est définie par un motif choisi parmi SEQ ID N°30 à SEQ ID N°40,
et en ce que ledit domaine peptidique comprend, entre l'extrémité N terminale et l'extrémité C terminale au moins un motif choisi parmi SEQ ID N°41, SEQ ID N°42 et SEQ ID NO : 73.
Par domaine peptidique selon l'invention, on entend une zone minimale de l'enveloppe d'un virus du groupe d'interférence HERV-W nécessaire à la reconnaissance d'un récepteur hASCT.
Les domaines peptidiques de l'invention peuvent être obtenus par la technique du génie génétique qui comprend les étapes de :
- culture d'un microorganisme ou de cellules eucaryotes transformé(es) à l'aide d'une séquence nucléotidique selon l'invention et
- récupération du domaine peptidique produit par ledit microorganisme ou lesdites cellules eucaryotes.
Cette technique est bien connue de l'homme du métier. Pour plus de détail la concernant, on pourra se référer à l'ouvrage ci-après : Recombinant DNA Technology I, Editors Ales Prokop, Raskesh K Bajpai ; Annals of the New-York Academy of Sciences, Volume 646, 1991. Les domaines peptidiques de l'invention peuvent également être préparés par les synthèses peptidiques classiques bien connues de l'homme du métier.
Par groupe d'interférence, on entend l'ensemble des virus dont l'infection (expression) d'une cellule par un de ses membres empêche l'infection par un autre membre du groupe par interférence de récepteur.
Par acide aminé quelconque, on entend notamment un acide aminé choisi parmi l'arginine, l'histidine, la lysine, l'acide aspartique, l'acide glutamique, l'asparagine, la glutamine, la sérine, la thréonine, l'asparagine, la thréonine, l'alanine, l'isoleucine, la leucine, la méthionine, la phénylalanine, le tryptophane, la tyrosine, la valine, la cystéine, la glycine, la proline.
Par récepteur hASCT, on entend tout transporteur d'acides aminés neutres sodium dépendant.
Par motifs, on entend une succession d'acides aminés correspondant à une région d'intérêt particulière du domaine peptidique selon l'invention, qui est exprimée par l'ensemble des virus du groupe d'interférence du virus HERV-W.
Préférentiellement, α est un nombre entier compris entre 3 et 18.
Préférentiellement, X ou Xaa du motif Pro Cys Xaa Cys dans les SEQ ID N°1 à SEQ ID N°29 est un acide aminé choisi parmi l'acide aspartique, l'acide glutamique, l'arginine : ces séquences sont ainsi de préférence choisies parmi SEQ ID NO : 44 à SEQ ID NO : 72.
Préférentiellement, Xₐ, X_{b}, X_{c} ou les acides aminés en position 3, 4 et 5 des SEQ ID N°s 30 à 40 sont une glycine, X_{d} ou l'acide aminé Xaa en position 6 des SEQ ID N°s 30 à 40 est un acide aminé choisi parmi la proline et la valine; Xₑ ou l'acide aminé Xaa en position 7 des des SEQ ID N°s 30 à 40 est un acide aminé choisi parmi la glutamine, la leucine et la thréonine; X_{f} ou l'acide aminé Xaa en position 9 des SEQ ID N°s 30 à 40 est un acide aminé choisi parmi la lysine, la thréonine, la méthionine et la glutamine, Xg ou l'acide aminé en position 10 des SEQ ID N°s 30 à 40 est un acide aminé choisi parmi l'alanine, la lysine, l'isoleucine, la thréonine et la valine.
Préférentiellement, β est un nombre entier égale à 20.
Préférentiellement, X₂ ou l'acide aminé Xaa en position 3 de SEQ ID N° 41 est un acide aminé choisi parmi l'asparagine, la thréonine, l'acide glutamique, l'histidine, X₃ ou l'acide aminé Xaa en position 4 de SEQ ID N° 41 est un acide aminé choisi parmi l'histidine, l'alanine, la serine, la lysine, l'acide glutamique; X₄ ou l'acide aminé Xaa en position 5 de SEQ ID N° 41 est un acide aminé choisi parmi la tyrosine, la thréonine, l'alanine, X₅ ou l'acide aminé Xaa en position 6 de SEQ ID N° 41 est un acide aminé choisi parmi la glutamine, l'arginine, la thréonine, X₆ ou l'acide aminé Xaa en position 7 de SEQ ID N° 41 est un acide aminé choisi parmi la leucine, la glutamine, l'acide glutamique.
Préférentiellement X₇ ou l'acide aminé Xaa en position 2 de SEQ ID N° 42 est un acide aminé choisi parmi la proline, la thréonine, l'arginine et l'asparagine; X₈ ou l'acide aminé Xaa en position 3 de SEQ ID N° 42 est un acide aminé choisi parmi la glycine, l'acide glutamique, l'asparagine, X₉ ou l'acide aminé Xaa en position 4 de SEQ ID N° 42 est un acide aminé choisi parmi la glycine, l'asparagine, l'isoleucine, la thréonine, la serine, X₁₀ ou l'acide aminé Xaa en position 5 de SEQ ID N° 42 est une lysine ou est délété ; X₁₁ ou l'acide aminé Xaa en position 6 de SEQ ID N° 42 est un acide aminé choisi parmi la lysine, la valine, l'isoleucine, la leucine, X₁₂ ou l'acide aminé Xaa, en position 7 de SEQ ID N° 42 est un acide aminé choisi parmi la glycine, l'asparagine; X₁₃ ou l'acide aminé Xaa en position 8 de SEQ ID N° 42 est un acide aminé choisi parmi la glutamine, la lysine, la valine, X₁₄ ou l'acide aminé Xaa en position 9 de SEQ ID N° 42 est un acide aminé choisi parmi la valine, la proline, la serine, la thréonine, X₁₅ ou l'acide aminé Xaa en position 10 de SEQ ID N° 42 est un acide aminé choisi parmi la valine, l'isoleucine.
Préférentiellement,
l'acide aminé Xaa en position 2 de SEQ ID NO : 73 est choisi parmi la Proline, la Thréonine, l'arginine et l'asparagine
l'acide aminé Xaa en position 3 de SEQ ID NO : 73 est choisi parmi la glycine,
l'acide glutamique, l'asparagine
l'acide aminé Xaa en position 4 de SEQ ID NO : 73 est choisi parmi la glycine, l'asparagine, l'isoleucine, la thréonine, la serine
l'acide aminé Xaa en position 5 de SEQ ID NO : 73 est choisi parmi la lysine, la valine, l'isoleucine, la leucine
l'acide aminé Xaa en position 6 de SEQ ID NO : 73 est choisi parmi la glycine, l'asparagine
l'acide aminé Xaa en position 7 de SEQ ID NO : 73 est choisi parmi la glutamine, la lysine, la valine
l'acide aminé Xaa en position 8 de SEQ ID NO : 73 est choisi parmi la valine, la proline, la sérine, la thréonine
l'acide aminé Xaa en position 9 de SEQ ID NO : 73 est choisi parmi la valine, l'isoleucine

Des délétions sont possibles dans les domaines selon l'invention indiqués ci dessus. Selon un mode particulier de réalisation de l'invention, X₁₀ est délété.

L'invention concerne également un domaine peptidique selon l'invention, caractérisé en ce qu'il induit une réponse immune contre un virus appartenant au groupe d'interférence de HERV-W, ledit domaine peptidique consistant en SEQ ID NO : 74.
Le domaine peptidique ainsi défini est reconnu par un récepteur situé à la surface d'un lymphocyte B ou T ou d'un anticorps circulant.
Par réponse immune, on entend l'ensemble des mécanismes biologiques permettant à un organisme pluricellulaire de maintenir la cohérence des cellules et tissus qui le constituent et d'assurer son intégrité en réponse à tout agression qui modifie les structures moléculaires de ses constituants ou qui introduit des molécules étrangères dans l'organisme.
Dans le contexte de l'invention, il est également décrit une séquence nucléotidique codant pour un domaine peptidique tel que défini ci dessus. De telles séquences peuvent être préparées par synthèse chimique et génie génétique en utilisant les techniques bien connues de l'homme du métier et décrites par exemple dans Sambrook J. et al., Molecular Cloning : A Laboratory Manual, 1989.
Dans le contexte de l'invention, il est également décrit un vecteur d'expression comprenant une séquence nucléotidique ci-dessus, ainsi que les moyens nécessaires à son expression.
A titre de vecteur d'expression, on peut citer par exemple les plasmides, les vecteurs viraux du type virus de la vaccine, adenovirus, baculovirus, poxvirus, rétrovirus, les vecteurs bactériens du type salmonelle, BCG.
Par moyens nécessaires à son expression, on entend tout moyen qui permet d'obtenir un peptide à partir d'une séquence nucléotidique, tel que notamment un promoteur, un terminateur de transcription, une origine de réplication et de préférence un marqueur de sélection.
Les vecteurs d'expression ci-dessus peuvent également comprendre des séquences nécessaires au ciblage des peptides vers des compartiments cellulaires particuliers.
Dans le contexte de l"invention, il est également décrit un microorganisme ou cellule hôte transformé par au moins un vecteur d'expression ci-dessus.
A titre d'exemples de microorganismes qui conviennent dans le contexte de l'invention, on peut citer les levures, telles que celles des familles suivantes : *Saccharomyces, Schizosaccharomyces, Kluveromyces, Pichia, Hanseluna, Yarowia, Schwaniomyces, Zygosaccharomyces, ; Saccharomyces cerevisiae, Saccharomyces carlsbergensis* et *Kluveromyces lactis* étant préférées ; et les bactéries, telles que *E. coli* et celles des familles suivantes : *Lactobacillus, Lactococcus, Salmonella, Streptococcus, Bacillus* et *Streptomyces.*
A titre d'exemples de cellules hôtes transformées, on peut citer les cellules provenant d'animaux tels que les mammifères, les reptiles, les insectes et équivalents. Les cellules eucaryotes préférées sont les cellules provenant du hamster chinois (cellules CHO), du singe (cellules COS et Vero), du rein de jeune hamster (cellules BHK), du rein de cochon (cellules PK 15) et du rein de lapin (cellules RK13, les lignées cellulaires humaines de l'ostéosacorme (cellules 143 B), les lignées cellulaires humaines HeLa et les lignées cellulaires humaines de l'hépatome (du type cellules Hep G2), ainsi que les lignées cellulaires d'insecte (par exemple de Spodoptera frugiperda), une lignée cellulaire de rein embryonnaire humaine (par exemple HEK293T). Les cellules hôtes peuvent être fournies dans des cultures en suspension ou en flacon, dans des cultures tissulaires, des cultures d'organe et équivalents.
L'invention concerne également un anticorps monoclonal dirigé contre un domaine peptidique selon l'invention.
Par anticorps, on entend tant un anticorps entier qu'un fragment d'anticorps.
Les anticorps recombinants peuvent être obtenus selon des procédés classiques connus de l'homme du métier, à partir d'organismes procaryotes, tels que bactéries, ou à partir d'organismes eucaryotes, tels que levures, cellules de mammifères, de plantes, d'insectes ou d'animaux, ou par des systèmes de production extra-cellulaire.
Les anticorps monoclonaux peuvent être préparés selon les techniques classiques connues de l'homme du métier telles que la technique des hybridomes dont le principe général est rappelé ci-après.
Dans un premier temps, on immunise un animal, généralement une souris, (ou des cellules en culture dans le cadre d'immunisations *in vitro)* avec un antigène cible d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité.
Par fragment d'anticorps, on entend tout fragment d'anticorps consécutif à une réponse immune contre un virus appartenant au groupe d'interférence de HERV-W. Ces fragments d'anticorps peuvent par exemple être obtenus par protéolyse. Ainsi, ils peuvent être obtenus par digestion enzymatique, résultant en des fragments de type Fab (traitement à la papaïne ; Porter RR, 1959, Biochem. J., 73 : 119-126) ou de type F(ab)'₂ (traitement à la pepsine ; Nisonoff A. et al., 1960, Science, 132 : 1770-1771). Ils peuvent également être préparés par voie recombinante (Skerra A., 1993, Curr. Opin. Immunol., 5 : 256-262). Un autre fragment d'anticorps qui convient aux fins de l'invention comprend un fragment Fv qui est un dimère constitué de l'association non covalente du domaine variable léger (VL) et du domaine variable lourd (VH) du fragment Fab, donc de l'association de deux chaînes polypeptidiques. Afin d'améliorer la stabilité du fragment Fv due à la dissociation des deux chaînes polypeptidiques, ce fragment Fv peut être modifié par génie génétique en insérant un lien peptidique adapté entre le domaine VL et le domaine VH (Huston P. et al., 1988, Proc. Natl.Acad. Sci.USA, 85 : 5879-5883). On parle alors de fragment scFv (« single chain Fragment variable ») car il est constitué d'une seule chaîne polypeptidique. L'utilisation d'un lien peptidique composé préférentiellement de 15 à 25 acides aminés permet de relier l'extrémité C-terminale d'un domaine à l'extrémité N-terminale de l'autre domaine, constituant ainsi une molécule monomérique dotée de propriétés de liaison similaires à celles de l'anticorps sous sa forme complète. Les deux orientations des domaines VL et VH conviennent (VL-lien-VH et VH-lien-VL) car elles présentent des propriétés fonctionnelles identiques. Bien entendu, tout fragment connu de l'homme du métier et présentant les caractéristiques immunologiques définies ci-dessus conviennent aux fins de l'invention.
L'invention concerne également l'utilisation d'au moins un domaine peptidique selon l'invention, ou d'au moins un anticorps selon l'invention pour la préparation d'un médicament destiné à l'inhibition, la prévention ou le traitement d'une infection provoquée par un virus appartenant au groupe d'interférence de
HERV-W chez un animal, de préférence l'homme. Le domaine peptidique selon l'invention peut notamment être utilisé pour cibler des cellules exprimant un récepteur de la famille hASCT afin de transduire un signal, moduler le flux des acides aminés (traitements du cancer).

Par éléments nécessaires à une expression constitutive des peptides, on entend un promoteur ubiquitaire ou spécifique des cellules eucaryotes. A titre d'éléments nécessaires à une expression inductible des peptides, on peut citer les éléments de régulation de l'opéron de *E. coli* pour la résistance à la tétracycline (Gossen M. et al, Proc Natl Acad Sci USA, 89 : 5547-5551 (1992).

L'utilisation d'au moins un domaine peptidique selon l'invention est particulièrement adaptée pour la préparation d'un médicament destiné à la prévention d'une infection provoquée par un virus appartenant au groupe d'interférence de HERV-W chez un animal, de préférence l'homme. L'utilisation d'au moins un anticorps selon l'invention est particulièrement adaptée pour la préparation d'un médicament destiné à l'inhibition ou le traitement d'une infection ou d'une pathologie induite par un virus appartenant au groupe d'interférence de HERV-W chez un animal, de préférence l'homme.

L'invention concerne également une composition pharmaceutique, comprenant à titre de substance active, au moins un domaine peptidique selon l'invention, , en association avec un véhicule pharmaceutiquement approprié. L'invention concerne également une composition pharmaceutique comprenant à titre de substance active, au moins un anticorps selon l'invention, en association avec un véhicule pharmaceutiquement approprié.
Bien entendu, l'homme du métier déterminera facilement le véhicule pharmaceutiquement approprié et la quantité de domaine peptidiques, d'épitopes, d'acides nucléotidiques ou d'anticorps à utiliser en fonction des constituants de la composition pharmaceutique.
Dans les compositions pharmaceutiques selon l'invention, pour l'administration orale, sublingale, sous cutanée, intra musculaire, intra veineuse, topique, intratrachéale, rectale, transdermique, la substance active peut être administrée sous formes unitaires d'administration ou en mélange avec des supports pharmaceutiques classiques, et destinés à une administration par voie orale, par exemple sous la forme d'un comprimé, d'une gélule, d'une solution buvable, etc..., ou par voie rectale, sous la forme d'un suppositoire, par voie parentérale, en particulier sous la forme d'une solution injectable, notamment par voie intraveineuse, intradermique, sous cutanée, etc..., selon des protocoles classiques bien connus de l'homme du métier. Pour l'application topique, on peut utiliser la substance active dans des crèmes, pommades, lotions, collyres.
Lorsqu'on prépare une composition solide sous forme de comprimés, on mélange la substance active avec un excipient pharmaceutiquement acceptable également nommé véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées. On peut également les traiter de telles sortes qu'ils aient une activité prolongée ou retardée et qu'ils libèrent d'une façon continue une quantité prédéterminée de la substance active. On peut également obtenir une préparation en gélules en mélangeant la substance active avec un diluant et en versant le mélange dans des gélules molles ou dures. On peut également obtenir une préparation sous forme de sirop ou pour l'administration sous forme de gouttes, dans laquelle la substance active est présente conjointement avec un édulcorant, un antiseptique, tel que notamment du méthylparaben et du propylparaben, ainsi qu'un agent donnant du goût ou un colorant approprié. Les poudres ou les granules dispersibles dans l'eau peuvent contenir la substance active en mélange avec des agents de dispersion ou des agents mouillants, ou des agents de mise en suspension, bien connus de l'homme du métier. Pour une administration parentérale, on utilise des suspensions aqueuses, des solutions salines isotoniques ou des solutions stériles et injectables qui contiennent des agents de dispersion, des mouillants pharmacologiquement compatibles, tels que notamment le propyléneglycol ou le butylèneglycol.
Le médicament ou la composition pharmaceutique selon l'invention peut comprendre en outre un agent d'activation qui induit les effets d'une médication ou renforce ou complète les effets de la médication principale, en augmentant notamment la biodisponibilité de la médication principale.
La posologie dépend de la gravité de l'affection, et sera adapté selon un protocole classique. A titre indicatif, lorsque la substance active est un anticorps monoclonal, la dose hebdomadaire est de 1 à 10 mg / kg, en combinaison avec un excipient pharmaceutiquement acceptable.
L'invention concerne également une composition diagnostique pour la détection et/ou la quantification d'un virus appartenant au groupe d'interférence de HERV-W ou la détection et/ou la quantification d'une réponse immune contre ledit virus, comprenant au moins un domaine peptidique selon l'invention ou au moins un anticorps selon l'invention.
Une composition diagnostique comprenant au moins un domaine peptidique selon l'invention est particulièrement adaptée si on souhaite déterminer si un patient présente une réponse immune contre un virus appartenant au groupe d'interférence de HERV-W alors qu'une composition diagnostique comprenant au moins un anticorps selon l'invention est particulièrement adaptée pour la détection et/ou la quantification d'un virus appartenant au groupe d'interférence de HERV-W. L'invention concerne également un procédé de détection et/ou de quantification d'un virus appartenant au groupe d'interférence de HERV-W dans un échantillon biologique prélevé chez un individu susceptible d'être infecté par ledit virus caractérisé en ce qu'il comprend les étapes consistant à :
- mettre en contact ledit échantillon biologique au moins un anticoprs selon l'invention dans des conditions permettant la formation d'un complexe entre le virus et l'anticorps, et
- détecter et/ou quantifier la formation dudit complexe par tout moyen approprié.
Par échantillon biologique, on entend un échantillon biologique d'origine humaine ou animale susceptible de contenir ledit virus tel qu'un échantillon de sang, de plasma, de sérum, d'urine, de liquide cephalo rachidien, ou de tissus tels que placenta, testicules, prostate, sein.
L'étape de mise en contact est une étape classiquement connue de l'homme du métier.
L'étape de détection / quantification peut être réalisée par tout moyen de détection connu dans le domaine des dosages immunologiques de très petites molécules, tel que la détection directe, c'est-à-dire sans l'intermédiaire de partenaire(s) de liaison, et la détection indirecte, c'est-à-dire par l'intermédiaire de partenaire(s) de liaison. La détection directe de la liaison entre l'anticorps ou fragment d'anticorps de l'invention et le virus peut être mise en oeuvre par exemple par résonance plasmonique de surface ou par voltamétrie cyclique sur une électrode portant un polymère conducteur. Dans ce cas, l'anticorps de l'invention sert à immunocapturer tout ou partie du virus, qui est ensuite éluée. L'élution peut être mise en oeuvre par tout procédé d'élution connu de l'homme du métier, tel qu'un choc de pH.
Dans le cas de la détection indirecte, la deuxième étape du procédé de l'invention peut être mise en oeuvre selon la technique classique ELISA de dosage par compétition. L'anticorps de l'invention sert alors de partenaire de liaison servant à la capture de tout ou partie du virus dans l'échantillon. La détection peut alors être mise en oeuvre par compétition entre tout ou partie du virus susceptible d'être contenue dans l'échantillon à tester et une quantité connue de virus préalablement marquée. Par marquage, on entend la fixation d'un marqueur capable de générer directement ou indirectement un signal détectable. Une liste non limitative de ces marqueurs consiste en :
▪ les enzymes qui produisent un signal détectable par exemple par colorimétrie, fluorescence, luminescence, comme la peroxydase de raifort, la phosphatase alcaline, l'acétylcholine estérase, la b-galactosidase, la glucose-6-phosphate déshydrogénase,
▪ les chromophores comme les composés luminescents, colorants,
▪ les molécules radioactives comme le 32P, le 35S ou le 125I,
▪ les molécules fluorescentes telles que la fluorescéine, la rhodomine, l'alexa ou les phycocyanines, et
▪ les particules telles que les particules en or, en latex magnétique, les liposomes.
Des systèmes indirects de marquage peuvent être aussi utilisés, comme par exemple par l'intermédiaire d'un autre couple ligand/anti-ligand. Les couples ligand/anti-ligand sont bien connus de l'homme du métier, et on peut citer par exemple les couples suivants: biotine/streptavidine, biotine/avidine, haptène/anticorps, antigène/anticorps, peptide/anticorps, sucre/lectine, polynucléotide/complémentaire du polynucléotide. Dans ce cas, c'est le ligand qui est lié au partenaire de liaison. L'anti-ligand peut être détectable directement par les marqueurs décrits au paragraphe précédent ou être lui-même détectable par un ligand/anti-ligand.
Ces systèmes indirects peuvent conduire, dans certaines conditions, à une amplification du signal. Cette technique d'amplification du signal est bien connue de l'homme du métier, et l'on pourra se reporter aux demandes de brevet antérieures FR98/10084 ou WO95/08000 de la Demanderesse ou à l'article J. Histochem. Cytochem., (1997), 45 : 481-491.
Le marquage de molécules est largement connu de l'homme du métier et est décrit par exemple par Greg T. Hermanson dans Bioconjugate Techniques, 1996, Academic Press Inc, 525B Street, San Diego, CA92101 USA.
Selon le type de marquage utilisé, comme par exemple en utilisant une enzyme, l'homme du métier ajoutera des réactifs permettant la visualisation du marquage.
De tels réactifs sont largement connus de l'homme du métier et sont décrits notamment dans Principles and Practice of Immunoessay, 2nd Edition, Edited by C. Price, D.J. Newman Stockton Press, 1997, 345 Park Avenue South, New York.
L'invention concerne également l'utilisation de la composition ci dessus pour le diagnostic *in vitro* d'un virus appartenant au groupe d'interférence de HERV-W dans un échantillon ou prélèvement biologique. En particulier, le diagnostic précoce d'un virus tel que le SRV1 et SRV2, virus impliqués dans des mécanismes d'immunodéficience chez le singe, permet de proposer un traitement adapté à l'hôte avant l'apparition d'une immunodéficience. Par ailleurs, le diagnostic précoce de HERV-W, impliqué dans des pathologies placentaires, permet de moduler son expression par exemple lors d'une préeclampsie. Dans le contexte de l'invention, il est également décrit l'utilisation d'un domaine peptidique selon l'invention ou d'un anticorps selon l'invention pour inhiber l'interaction entre l'enveloppe d'un virus appartenant au groupe d'interférence de HERV-W et un récepteur ASCT. Ceci permet notamment d'obtenir une immunothérapie contraceptive.
L'invention concerne également l'utilisation d'un domaine peptidique selon l'invention pour identifier des molécules chimiques ou biologiques dont l'interaction avec tout ou partie de ce domaine peptidique, bloque l'interaction entre l'enveloppe d'un virus appartenant au groupe d'interférence de HERV-W et un récepteur ASCT. Par exemple, lorsqu'on utilise un domaine peptidique selon l'invention de HERV-W, ceci permet d'obtenir notamment des molécules chimiques ou biologiques très adaptées pour obtenir un traitement contraceptif. L'utilisation de telles molécules chimiques pour inhiber l'interaction entre l'enveloppe d'un virus appartenant au groupe d'interférence de HERV-W et un récepteur ASCT présente un intérêt thérapeutique.
A titre indicatif, deux procédés génériques permettant le criblage des molécules chimiques ou biologiques susceptibles d'inhiber l'interaction env / récepteur sont décrits ci dessous.
Dans un contexte où il est possible de produire une enveloppe soluble, il convient de déterminer si une molécule chimique ou biologique altère l'interaction env/récepteur selon une méthode de type ELISA utilisant des cellules exprimant au moins un récepteur hASCT en phase de capture. Ainsi, sur une plaque de 96 puits, des cellules exprimant un récepteur hASCT d'intérêt sont cultivées ou adsorbées, et une interaction env / récepteur est détectée via l'utilisation d'une enveloppe soluble marquée (tag histine, fusion GPF), et donc capable de générer un signal de référence dosable. Si après préincubation de ladite enveloppe soluble avec une molécule chimique ou biologique, une réduction du signal est observé, cela signifie que la molécule chimique ou biologique altère l'interaction env/récepteur. Alternativement, il est possible d'utiliser un vecteur rétroviral pseudotypé par l'enveloppe d'intérêt et exprimant un marqueur détectable (LacZ) et procéder au même test. Il est également possible de sélectionner des molécules d'intérêts via une mesure d'inhibition de fusion. Des cellules exprimant le récepteur d'intérêt (cell-récept), par exemple des cellules HeLa ou XC-RDR ainsi que des cellules exprimant constitutivement un marqueur (par exemple lacZ) ainsi que transitoirement ou de façon stable l'enveloppe d'intérêt (cell-env-LacZ) sont utilisées; l'enveloppe d'intérêt a été au préalable modifiée au niveau de sa queue intracytoplasmique par échange avec le domaine intracytoplasmique de env HERV-W afin de la rendre constitutivement fusogène (Cheynet et al, 79(9): 5586-5593, 2005). La mise en présence des deux types cellulaires, "cell-récept" en excès et "cell-env-LacZ" en défaut, conduit à la formation de cellules multinuclées génates ou syncytia, contenant 1 ou 2 noyaux bleus venant de"cell-env-LacZ" et des dizaines de noyaux blancs venant de "cell-récept". Une même co-culture réalisée en présence de molécule chimique ou biologique altérant l'interaction env/récepteur conduit à une diminution du nombre de syncytia et à leur contenu en noyau. Une automatisation de telles mesures à l'aide d'une caméra ccd est possible.

Dans le contexte de l'invention, on décrit également l'utilisation d'un domaine peptidique selon l'invention pour générer des anticorps bloquant l'interaction entre l'enveloppe d'un virus appartenant au groupe d'interférence de HERV-W et un récepteur hASCT.
Il est également décrit un procédé pour déterminer une région polypeptidique nécessaire à l'interaction entre l'enveloppe d'un virus appartenant au groupe d'interférence de HERV-W et un récepteur hASCT caractérisé en ce que :
▪ on identifie la séquence nucléotidique et/ou peptidique de l'enveloppe précurseur dudit virus
▪ on exclut la partie signal
▪ on détecte un domaine Serine-Acide Aspartique-Xₐ-X_{b}-X_{c}-X_{d}-Xₑ-Acide Aspartique-X_{f}-X_{g}
   dans lequel
   Xₐ, X_{b}, X_{c}, X_{d}, Xₑ, X_{f}, X_{g}, sont des acides aminés quelconque qui correspond à la SED ID N°43
▪ on exclut de l'extrémité C-terminale entre 15 et 25 acides aminés, préférentiellement 20 acides aminés, après ledit domaine Serine-Acide aspartique-Xₐ-X_{b}-X_{c}-X_{d}-Xₑ-Acide aspartique -X_{f}-X_{g}, qui correspond à la SED ID N°43
Préférentiellement Xₐ, X_{b}, X_{c} est un acide aminé qui est la glycine, X_{d} est une acide aminé choisi parmi la proline et la valine; Xₑ est un acide aminé choisi parmi la glutamine, la leucine, la thréonine ; X_{f} est un acide aminé choisi parmi la lysine, la thréonine, la méthionine, la glutamine, X_{g} est un acide aminé choisi parmi l'alanine, la lysine, l'isoleucine, la thréonine, la valine.
Au sens de la présente invention, on identifie la séquence nucléotidique et/ou conséquent peptidique de l'enveloppe précurseur dudit virus par tout moyen connu de l'homme du métier, qui pourra notamment se référer au Maniatis (Ed 1989).
On exclut la partie signal par tout moyen connu de l'homme du métier tel que décrit notamment dans « Improved Prédiction of Signal Peptide: SignalP 3.0 » Jannick Dyrløv Bendtsen, Henrik Nielsen, Gunnar von Heijne and Søren Brunak.J. Mol. Biol., 340:783-795, 2004.

On détecte ledit domaine par tout moyen connu de l'homme du métier, c'est à dire en utilisant un logiciel de type Blast ou Fasta (voir notamment Altschul SF, Gish W, Miller W, Myers EW, Lipman DJ., Basic local alignment search tool. J Mol Biol. 1990 Oct 5;215(3):403-10).

Les figures ci-jointes sont données à titre d'exemple explicatif et n'ont aucun caractère limitatif. Elles permettront de mieux comprendre l'invention.

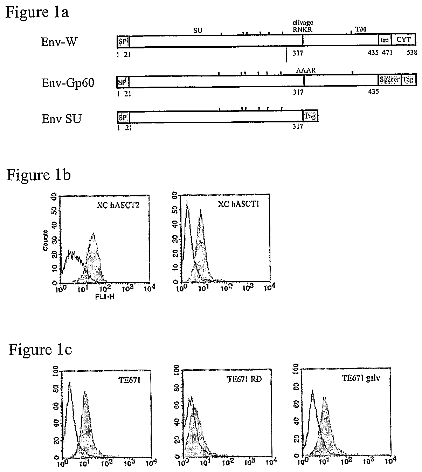

La figure 1 illustre les caractéristiques et propriétés phénotypiques de protéines recombinantes solubles dérivées de Env-W. Notamment, la figure 1a illustre la protéine recombinante soluble la plus grande englobant tout ou partie des sous-unités SU et TM (Env-Gp60) et la protéine recombinante soluble correspondant à la sous-unité SU (EnvSU). La figure 1b représente l'analyse par cytométrie de flux du test de fixation de la protéine recombinante EnvSU sur les cellules XC hASCT2 et XC hASCT1 exprimant respectivement les récepteurs hASCT2 et hASCT1. La figure 1c illustre le test d'interférence de fixation sur les cellules TE671 (contrôle hASCT2), TE671RD (hASCT2 bloqué) et TE671galv (Pit1 bloqué).

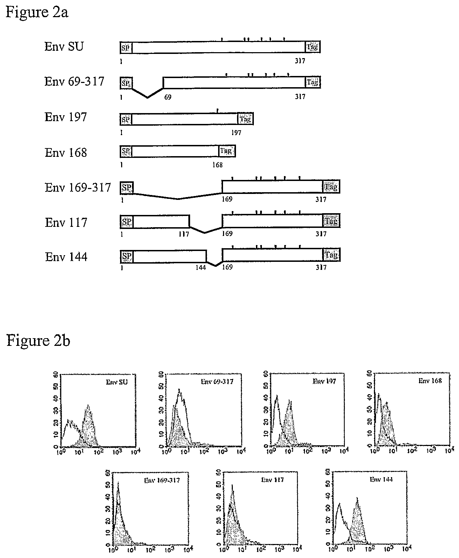

La figure 2 illustre la définition du domaine minimum de fixation de l'enveloppe ERV-W sur le récepteur hASCT2 (RBD pour receptor binding domain). Notamment, la figure 2a décrit l'ensemble des mutants de délétion conçus à partir de EnvSU. La figure 2b représente l'analyse par cytométrie de flux du test de fixation des protéines recombinantes dérivées de EnvSU sur les cellules XC hASCT2 exprimant le récepteur hASCT2, en particulier la fixation des mutants Env197, Env168 et Env144 et le défaut de fixation des mutants Env69-317, Env169-317 et Env117.

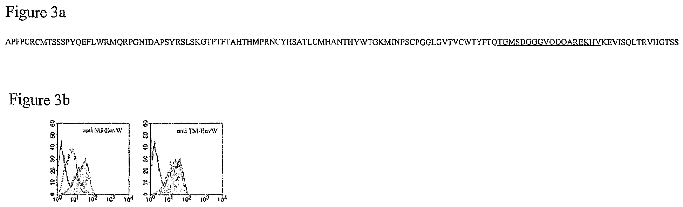

La figure 3a illustre la définition d'un peptide immunogène à l'intérieur du domaine selon l'invention (RBD) correspondant à la région 21-144 du précurseur de la protéine d'enveloppe d'HERV-W et identifié par SEQ ID NO : 74. La figure 3b montre l'inhibition de la fixation du RBD sur son récepteur à l'aide d'un anticorps produit à partir du peptide immunogène (antiSU-EnvW) et l'absence d'inhibition de la liaison RBD-récepteur en présence d'un anticorps non-spécifique (antiTM-EnvW).

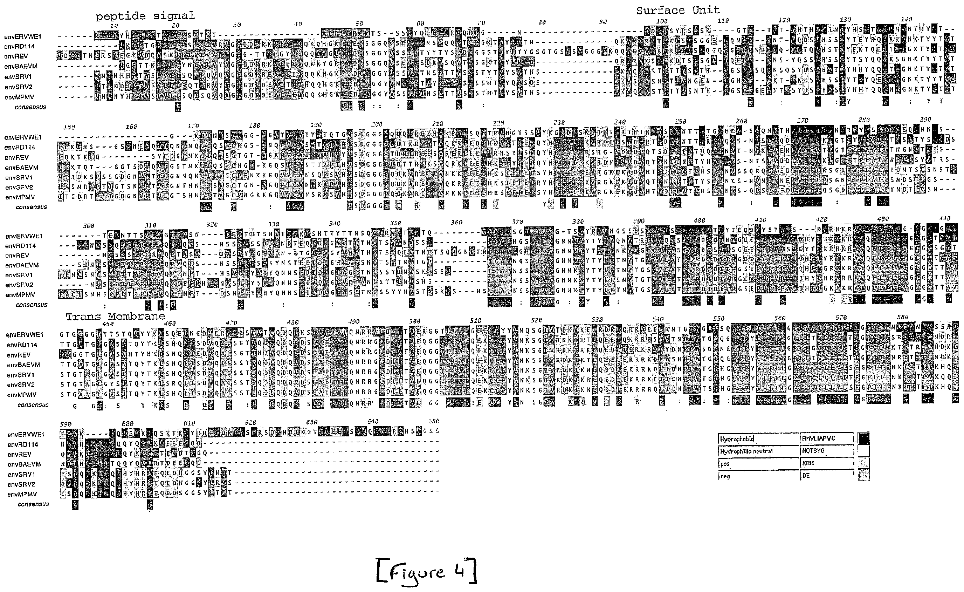

La figure 4 représente l'alignement des séquences rétrovirales d'enveloppes appartenant au même groupe d'interférence et présente les bornes du peptide signal, de la sous-unité SU (surface unit) et de la sous-unité TM (Trans membrane) ainsi que le site de fixation au récepteur. Les séquences sont HERV-W (Human Endogenous Retroviral Family W), RD114 (Cat Endogenous retrovirus), REV (Avian Reticuloendotheliosis Virus), BAEV (Baboon endogenous virus (strain M7)), SRV1 (Simian retrovirus SRV-1), SRV2 (Simian retrovirus SRV-2) et MPMV (Simian Mason-Pfizer virus).

Les exemples suivants sont donnés à titre illustratif et n'ont aucun caractère limitatif. Ils permettront de mieux comprendre l'invention.

### Exemple 1: caractérisation moléculaire et phénotypique d'enveloppes recombinantes

### Construction et production de la sous-unité SU d'enveloppe HERV-W.

A partir du vecteur d'expression phCMV-Env-W (Blond J Virol, Vol 74(7) :3321-3329, 2000) contenant le gène de l'enveloppe HERV-W (538 acides aminés) (clone PH74, Blond et al J Virol Vol 73(2) :1175-1185, 1999), un vecteur phCMVEnv-Gp60 permettant l'expression d'une protéine d'enveloppe recombinante soluble a été conçu.
L'enveloppe soluble (Gp60,1-435) a été construite comme décrit ci dessous:
(1) le site de clivage natif RNKR (AA 314 à 317) entre les sous-unités SU et TM a été muté en AAAR, afin de permettre la production d'une protéine de fusion stable et non de deux sous-unités SU-TM clivées puis ré-associées par un pont disulfure.
(2) Les régions transmembranaire (tm) et intracytoplasmique (CYT) correspondant aux acides aminés 436 à 538 on été supprimées afin d'obtenir une protéine soluble.
(3) Un bras espaceur de composition (GGGS)3 suivi d'une queue polyhistidine (RGS-HHHHHH) ont été ajoutés en position C-terminale, afin de permettre la purification de cette protéine par IMAC et la détection par un anticorps monoclonal anti-histidine (Qiagen, RGS H6).

A partir du vecteur phCMVEnv-Gp60 exprimant l'enveloppe soluble, le vecteur phCMV-EnvSU a été construit, permettant la production d'une protéine SU. La SU soluble est une protéine de fusion contenant une queue polyhistidine C-terminale de séquence RGS-HHHHHH immédiatement en aval de la séquence AAAR, afin de permettre la purification de cette protéine par IMAC et la détection par un anticorps monoclonal anti-histidine (Qiagen, RGS H6).

La structure schématique des différentes protéines produites à partir des vecteur phCMV-Env-W, phCMV-EnvGp60 et phCMV-EnvSU est illustrée en figure 1a.

### Production de l'enveloppe soluble.

Le plasmide d'expression phCMV-EnvGp60 ou phCMV-EnvSU est transfecté dans les cellules HEK293T par précipitation au phosphate de calcium. Le surnageant contenant l'enveloppe GP60 ou SU est collecté après 48 heures de production dans un milieu sans sérum et filtré sur des membranes 0,45µm pour éliminer les débris cellulaires. 20µl de surnageant sont directement analysés sur gel de polyacrylamide et par western blot avec un anticorps monoclonal anti-histidine (Qiagen, RGS H6). Les protéines GP60 et SU sont correctement exprimées sous forme soluble.

### Test de fixation et analyse par cytométrie en flux

Les lignées stables XChASCT2 et XChASCT1 exprimant constitutivement les récepteurs hASCT2 (XChASCT2) ou hASCT1 (XChASCT1) ont été établies après transfection des cellules XC (sarcome de rat) par des vecteurs exprimant l'un ou l'autre récepteur humain hASCT puis sélection d'un clone comme précédemment décrit (Frendo et al, Mol Cell Biol, Vol 23(10) : 3566-3574, 2003). Les cellules humaines suivantes sont décrites dans Blond J Virol, Vol 74(7) :3321-3329, 2000. Les cellules TE671 expriment hASCT2. Les cellules TE671RD expriment constitutivement l'enveloppe RD114 (retrovirus endogène de chat) appartenant au même groupe d'interférence et reconnaissant donc le récepteur hASCT2. Les TE671galv expriment constitutivement l'enveloppe GALV (gibbon ape leukemia virus) appartenant à un autre groupe d'interférence et reconnaissant le récepteur PiT1.

Les cellules ont été lavées dans du PBS et récoltées par décollement avec 0,02% de versene en PBS. Un total de 10⁶ cellules a été incubé avec 1 ml de surnageant filtré contenant l'enveloppe soluble (Gp60 ou SU) pendant 1 heure à 37°C. Les cellules ont été lavées avec du PBA (PBS et 0,5% de sodium d'azide) contenant 2% de sérum de veau foetal et ont été marquées pendant 1 heure à 4°C avec un anticorps monoclonal anti-histidine (RGSH6, Qiagen). Les cellules ont été lavées une fois avec du PBA et incubées avec un anticorps secondaire couplé à l'isothiocyanate de fluoresceine pendant 1 heure à 4°C. Les cellules ont été lavées deux fois avec du PBA et analysées par cytométrie en flux.

En utilisant les cellules cibles XChASCT2, les inventeurs ont mis en évidence que la protéine recombinante Gp60 correspondant à une forme soluble de l'enveloppe présente une caractéristique phénotypique identique à celle de l'enveloppe sauvage, à savoir qu'elle est capable de se fixer sur les cellules XC exprimant le récepteur hASCT2.

En utilisant les cellules cibles XChASCT2, XChASCT1, TE671, TE671RD, TE671 galv, les inventeurs ont mis en évidence que la protéine recombinante correspondant à la sous-unité SU de l'enveloppe présente des caractéristiques phénotypiques identiques à celles de l'enveloppe sauvage. Tout d'abord, la sous-unité SU est capable de se fixer aux deux récepteurs hASCT1 et hASCT2 (Figure 1b). De plus, cette protéine a été testée vis à vis de cellules humaines TE671 et de cellules dérivées TE671RD et TE671galv. La protéine SU soluble se fixait sur les cellules TE671 exprimant le récepteur hASCT2 et les cellules TE671galv bloquées pour le récepteur PiT1, mais ne se fixait pas sur les cellules TE671RD bloquées pour le récepteur hASCT2 (Figure1c). La protéine recombinante SU et l'enveloppe du retrovirus RD 114 interféraient spécifiquement.

### Exemple 2 : Identification des domaines d'interaction de la partie SU de l'enveloppe W avec son récepteur hASCT2

Afin d'identifier les bornes de la région de l'enveloppe se fixant au récepteur hASCT2, les inventeurs ont construit un ensemble de mutants de délétion à partir des extrémités N- et C- terminales. Les domaines de la sous-unité SU ont été obtenus par PCR et sous-clonés dans le vecteur d'expression pHCMV-EnvSU et séquencés. Les plasmides d'expression phCMV-EnvSU, Env69-317, Env197, Env168, Env169-317, Env117 et Env144 (Figure 2a) ont été transfectés dans les cellules HEK293T par précipitation au phosphate de calcium. Les conditions d'obtention et d'analyse des protéines sont identiques à celles détaillées dans l'exemple 1.

Les protéines EnvSU, Env69-317, Env197 étaient correctement exprimées sous forme soluble. En utilisant les cellules XC exprimant constitutivement hASCT2, les inventeurs ont démontré que la protéine Env197 était capable de se fixer au récepteur exprimé à la surface des cellules comme la sous-unité SU (1-317). Ainsi, les 176 premiers résidus de la sous-unité SU mature (donc dépourvue de son peptide signal) étaient suffisants pour se fixer à la surface des cellules exprimant le récepteur hASCT2. La délétion de la région 21-68 entraînait une perte de la fixation au récepteur indiquant aussi son implication dans le domaine de fixation au récepteur (RBD pour receptor binding domain). Par contre, la protéine tronquée Env168 montrait une capacité de fixation au récepteur hASCT2 plus faible.
Afin d'obtenir des quantités équivalentes dans les surnageants entre les différentes protéines tronquées, les inventeurs ont fusionné deux plus petits domaines de la région N-terminale de la SU (Env117 et Env144) à la région C-terminale de la sous-unité SU (Env169-317), ce dernier domaine ne se fixant pas à hASCT2. Le niveau d'expression des protéines Env 117 et Env 144 était similaire et les protéines étaient exprimées sous forme soluble. Le test de fixation montrait que seule la protéine Env144 était capable de se fixer sur les cellules exprimant le récepteur hASCT2. L'absence de fixation de la protéine Env117 à la surface des cellules indiquait la perte d'au moins un déterminant de fixation à l'intérieur de la région 117-144.

Par conséquent, les bornes des domaines d'interaction de l'enveloppe W avec son récepteur sont définies par les acides aminés 21 à 144.

A noter que d'une manière générale, les protéines (y compris les protéines d'enveloppe) destinées à la sécrétion ou à l'expression membranaires sont synthétisées au niveau du réticulum endoplasmique granuleux (RE). La translocation des protéines néosynthétisées dans le RE est conditionnée par un peptide signal N-terminal (Walter et Lingappa 1986). La région hydrophobe du peptide signal initie la pénétration dans la membrane du réticulum, entraînant à sa suite le reste du peptide néosynthétisé. La translocation commençant en même temps que la synthèse, c'est le peptide en cours de traduction qui traverse la membrane du RE. Pendant que la protéine passe dans la lumière du RE, la séquence signal est clivée par une enzyme cellulaire spécifique, la signal peptidase (Walter P, Johnson AE: Signal sequence récognition and protein targeting to the endoplasmic reticulum membrane. Annu Rev Cell Biol 1994, 10:87-119.). La translocation de Env dans le RE est stoppée par le domaine transmembranaire (hydrophobe) de la glycoprotéine qui s'ancre aux membranes phospolipidiques. Dans la lumière du RE, les régions (SU et partie de TM) destinées à devenir extracytoplasmique sont repliées (les ponts disulfure formés), glycosylées et oligomérisées. Après oligomérisation, les protéines en cours de maturation sont transportées dans l'appareil de Golgi où elles subissent de nouveaux processus de maturation des glycanes ainsi que le clivage par des endoprotéases de type furine reconnaissant un motif R/KXXR conduisant aux deux sous-unités SU et TM.
La protéine mature est adressée à la membrane plasmique grâce à un motif présent sur la queue intracytoplasmique contenant une tyrosine (Y-X-X)aliphatique/aromatique)

### Exemple 3 : Test d'inhibition in vitro de la fixation de l'enveloppe sur son récepteur (définition d'un peptide et génération d'un anticorps de lapin)

A partir de la région définie dans l'exemple 2 et d'une détermination des régions potentiellement antigéniques de la sous-unité SU, un peptide (112-129, TGMSDGGGVQDQAREKHV+C, 19 acides aminés) a été défini. Une cystéine a été ajoutée en position C-terminale pour le couplage KLH (keyhole limpet hemocyanin, cf Frendo et al, Mol Cell Biol, Vol 23(10) : 3566-3574, 2003). Ce peptide a été utilisé pour immuniser un lapin puis pour purifier par affinité l'anticorps polyclonal dirigé contre la région 112-119 contenu dans le sérum de ce lapin.

La protéine Env144 est pré-incubée à 37°C pendant une heure avec soit l'anticorps polyclonal anti-SU soit avec un anti-TM. La formation du complexe protéine Env144-anticorps anti-SU réduisait drastiquement la fixation de l'enveloppe sur les cellules exprimant le récepteur hASCT2. Au contraire, l'utilisation d'un anticorps non dirigé contre le rbd n'altérait pas la fixation de celui-ci sur le récepteur hASCT2.

### Exemple 4: Obtention d'anticorps monoclonaux dirigés contre la protéine d'enveloppe de HERV-W.

### Immunisation des souris par ADN.

Trois souris BALB/c femelles de six semaines (IFFA-Credo) ont été immunisées par injection directe d'ADN plasmidique nu (phCMV-env-W) contenant le gène de l'enveloppe HERV-W. Les injections ont été réalisées par voie intradermique à l'aide d'un pistolet (« gene gun »). Cinq injections de 2 µg d'ADN ont d'abord été effectuées pour chaque souris suivies d'un rappel avec deux injections de 4 µg d'ADN. Les sérums ont été prélevés et le titre en anticorps pour chaque sérum a été déterminé. Le titre en anticorps étant trop faible, un lysat cellulaire a été préparé.Préparation du lysat cellulaire.

Les cellules de rhabdomyosarcome TelCeB6 (ATCC CRL8805) ont été transfectées par le plasmide phCMV-env-W. Après une vingtaine d'heures et présence de syncytia, un extrait cellulaire a été réalisé en tampon PBS 0,5% Triton. Les extraits protéiques ont été dosés par Bradford. La concentration en antigène env-W correspondait à 9,5 µg/µl de protéines totales.

### Immunisation des souris par extrait de lysat cellulaire.

Les mêmes souris ont tout d'abord reçu une injection de 10 µg de lysat cellulaire par voie intrapéritonéale suivie d'une injection de rappel de 2 x 100 µg de lysat cellulaire par voie intrapéritonéale. Trois jours avant la fusion avec les cellules de myélome une nouvelle injection a été effectuée, par voie intra-veineuse, de 22 µg de la protéine d'enveloppe soluble Gp60 obtenue à partir du plasmide phCMV-Env-Gp60 comme décrit à l'exemple 1 préalablement purifiée, avant injection, sur résine Ni-NTA (Qiagen) selon les conditions suivantes : fixation en tampon phosphate pH 8, lavages en tampon phosphate pH 8 et en acétate d'ammonium pH 6, élution en tampon d'acétate d'ammonium pH 3,5 et concentration par speed vac. 47 µg de la protéine Gp60 eucaryote ainsi obtenue ont été réservés pour l'injection par voie intra-veineuse décrite ci-dessus. Après fusion, les surnageants des hybridomes ont été testés par immunofluorescence sur les cellules transfectées et fixées (TeLCeb6), les anticorps ont été criblés par un test fonctionnel en ELISA en utilisant la protéine Env-W à une concentration de 9,2 µg/µl de protéines totales et une protéine Env AS comme contrôle négatif à une concentration de 13,3 µg/µl de protéines totales et les anticorps les plus performants ont été sélectionnés.

Les anticorps monoclonaux 2H1H8, 12C7A3 et 1F11B10 ont ainsi été obtenus. Les anticorps monoclonaux 2H1H8 et 12C7A3 sont dirigés contre la partie N-terminale non glycosylée de la région SU de la protéine Env-HERV-W. Ils sont dirigés contre le rdb comme montré par un essai en Western blot à l'aide de Env 144. L'anticorps monoclonal 1F11B10 est dirigé contre la partie C-terminale glycosylée de la région SU de la protéine Env-HERV-W comme montré par un essai en Western blot à l'aide de Env 169-317. Il ne reconnaît pas Env 144.

### Exemple 5: Test d'inhibition in vitro de la fixation de l'enveloppe sur son récepteur et d'inhibition de la formation de syncytia à l'aide d'anticorps monoclonaux par test de fusion de cellule à cellule (coculture).

Le plasmide d'expression de la glycoprotéine d'enveloppe est transfecté dans les cellules TELCeB6 par précipitation au phosphate de calcium à deux quantités 100 et 500ng (Cosset et al., Journal of Virology, 69 (10) : 6314-6322 (1995)). Les cellules exprimant l'enveloppe sont décrochées du support, 20 heures après tranfection et sont pré-incubées à 37°C pendant une heure, respectivement, avec l'anticorps monoclonal anti-HIV 23A5, l'anticorps monoclonal anti-TM Env-HERV-W 6A2B2 (obtenu antérieurement), les anticorps monoclonaux anti-SU Env-HERV-W 2H1H8 12C7A3 et 1F11B10 (dilution 1/50^{ème}). Puis, elles sont ré-ensemencées à concentration égale (0,4 x 10⁵ cellules/puits) en plaques 12 puits. Des cellules humaines indicatrices de carcinome épithélioïde (Hela, ATCC CCL-2,) sont alors ajoutées aux cellules transfectées à raison de 2 10⁵ cellules par puits et la co-culture est poursuivie pendant 24 h. Une coloration XGal (5-bromo-4chloro-3-indolyl-β-D-galactopyranoside peut alors être effectuée pour colorer le noyau des cellules TELCeB6 (Cosset et al., Journal of Virology, 69 (10) : 6314-6322 (1995)). Elle est suivie d'une coloration par des solutions de May-Grünwald et Giemsa (MERCK) effectuée selon les recommandations du fournisseur. La fusion observée correspond à une fusion " from within ", c'est-à-dire à une fusion de cellule-à-cellule, à partir d'une cellule exprimant l'enveloppe, par opposition à une fusion " from without " qui correspond à une formation de syncytia consécutive à une fusion virion-cellule(s). Les résultats présentés dans le tableau 1 ci-dessous expriment le nombre de cellules fusionnées comptées.

**Tableau 1**

| Anticorps | 23A5 | 6A2B2 | 2H1H8 | 1F11B10 | 12C7A3 |
|---|---|---|---|---|---|
| 100 ng | 261 | 231 | 130 | 223 | ND |
| 500 ng | 217 | 273 | 73 | 210 | 11 |

| | | | | | |
|---|---|---|---|---|---|
| ND : non déterminé | | | | | |

Les résultats présentés ci-dessus montrent que la formation du complexe protéique Env-anticorps anti-SU 2H1H8 et 12C7A3 réduisent drastiquement la fixation de l'enveloppe sur les cellules exprimant le récepteur hASCT2 ainsi que la fusion des cellules. Au contraire, l'utilisation d'un anticorps non dirigé contre le rdb (6A2B2 ou 1F11B10) n'altère pas la fixation de l'enveloppe et la fusion des cellules de manière significative, comme on peut le constater par comparaison aux résultats obtenus avec l'anticorps de contrôle anti-HIV 23A5.

### Exemple 6 : Etude de l'interaction in vivo entre la protéine d'enveloppe et le récepteur hASCT2 et de la formation in vivo de syncytia.

Pour vérifier les résultats obtenus *in vitro* un modèle animal a été conçu. Des cellules de rhabdomyosarcome TelCeB6 (ATCC CRL8805), en culture dans le milieu DMEM (Gibco Invitrogen 41966-029) complémenté avec du sérum d'Amérique du Sud, ont été respectivement transfectées, à l'aide du kit LipofectAMINE PLUS™ (Gibco Invitrogen), avec de l'ADN correspondant au gène *env* HERV-W cloné en sens à la concentration de 2 µg/µl (ADN 409), avec de l'ADN correspondant au gène *env* HERV-W cloné en anti-sens à la concentration de 1,5 µg/µl (ADN 410) et avec de l'ADN correspondant à un gène *env* HERV-W muté à la concentration de 1,3 µg/µl (ADN LQMV) selon le protocole détaillé ci-dessous. Les cellules transfectées par l'ADN 409 sont capables d'exprimer la protéine d'enveloppe W fusogène, les cellules transfectées par l'ADN 410 ne sont pas capables d'exprimer la protéine d'enveloppe et les cellules transfectées par l'ADN LQMV sont capables d'exprimer une protéine d'enveloppe W mutée, non fusogène.

### 1). Protocole :

### 1^{er} Jour : Mise en culture des cellules TelCeB6 :

- Inoculation des boîtes de 100mm de diamètre **→** 50-70% de confluence ;
- Incubation en milieu DMEM complémenté (6 ml par boîte) pendant 24 heures à 37°C sous 5% de CO₂.

### 2^{ème} Jour : Transfection avec le kit LipofectAMINE PLUS™ :

### 1 Précomplexation de l'ADN

- Mélange de 750µl de milieu non-complémenté avec les ADNs précités dans un tube falcon de 15 ml (référence 2096), soit 2µl de 409 ou 3µl de 410 ou 3µl LQMV ;
- Agitation sous Vortex du PLUS Reagent et en ajouter 20µl à la solution d'ADN ;
- Agitation sous Vortex immédiatement 10 secondes à 1400 tpm.
- Incubation 15 minutes à T° ambiante.

### 2 Préparation des cellules

- Remplacement par 5ml de milieu non-complémenté.

### 3 Dilution de la Lipofectamine

Dans un tube et pour une boîte, mélanger 30 µl de LipofectAMINE Reagent avec 750 µl de milieu non-complémenté.

### 4 Complexation de l'ADN

Mélange des 780 µl de Lipofectamine diluée et des 772 µl de solution d'ADN pré-complexé (total : 1552 µl) ;
Agitation sous Vortex immédiatement 10 sec à 1400 tpm,
Incubation 15 minutes à température ambiante.

### 5 Transfection et obtention d'animaux receveurs greffés avec les cellules cibles, traités ou non par injection d'anticorps anti-Env

Dépose des 1552 *µl* dans une boîte ;
Incubation 2-3 heures à 37°C sous 5% CO_{2;}
Remplacement du milieu de transfection par 6ml de milieu complémenté ; Incubation 1heure à 37°C sous 5% CO_{2;}

Injection par voie intrapéritonéale (IP) aux souris SCID (sous un volume de 1 ml), 1/5^{ème} de chaque boîte à 70% de confluence, avec ou sans injection supplémentaire d'anticorps anti-protéine Env (anticorps monoclonal 2H1H8, anticorps polyclonaux 69 (anti-SU) et 71 (anti-TM) au 1/100).

Obtention d'animaux tolérant la greffe et permettant la dissémination des cellules greffées dans l'organisme, parallèlement à l'établissement d'une pseudo-ascite dans la cavité péritonéale.

### 3^{ème} Jour :

Prélèvement des cellules de chaque animal par lavage péritonéal: °injection de 2 ml d'air suivie de 2 ml de sérum physiologique puis massage et récupération des 2 ml de liquide péritonéal (protocole mis au point pour la récupération chez l'animal greffé des cellules implantées dans la cavité péritonéale) ;
Observation au microscope « phase inverse » avec comptage des syncytia et/ou après coloration sur lame ;
Lecture est immédiate effectuée après étalement sur des lames à chambre quadrillée en présence de bleu Trypan (exclusion des cellules mortes). On dénombre le nombre de cellules ayant fusionnées entre elles par champ quadrillé avec un objectif « grand champ » (40) permettant d'établir le comptage sur plus d'une centaine de cellules afin de disposer de séries de comptage statistiquement représentatives.

Un aliquot cellulaire de chaque prélèvement est fixé en présence de méthanol/acétone (v/v) puis conservé à -20°C jusqu'à coloration au cristal violet (1%). Les lames colorées ont fait l'objet de prises photographiques.

### 2) Souris :

des lots de 2 souris sont inoculés avec :
les cellules transfectées avec les trois types de plasmide (ADNs 409, 410 et LQMV) sans anticorps (3x2=6 souris)
les cellules transfectées avec les trois types de plasmide (ADNs 409, 410 et LQMV) avec l'anticorps monoclonal 2H1H8 (3x2=6 souris)

### 3) Résultats :

Le nombre de cellules fusionnées est déterminé par lecture directe sur chambre de numération quadrillée pour 100 cellules est indiqué dans le tableau 2 ci-dessous :

**Tableau 2**

| Lecture ECP (bleu Tryptan) : lecture directe des syncytia | | | |
|---|---|---|---|
| Lignées | Numération S1 * | Numération S2^{*} | Moyenne |
| 409 contrôle | 19 | 22 | 20,5 |
| 409 + 2H1H8 | 3 | 4 | 3,5 |
| 410 contrôle | 8 | 11 | 9,5 |
| 410 + 2H1H8 | 2 | 3 | 2,5 |
| LQMV contrôle | 8 | 5 | 6,5 |
| LQMV + 2H1H8 | 1 | 1 | 1 |

| | | | |
|---|---|---|---|
| S1* et S2* = souris 1 et souris 2 | | | |

Chaque chiffre représente le nombre de cellules fusionnées et visualisées par champ étudié. Certaines cellules pouvant être superposées dans le trajet optique, le compte des cellules apparaissant fusionnées dans les contrôles est supérieur à zéro. La réalité des syncytia et la discrimination avec des empilements de cellules ont ensuite été vérifiées par coloration des cellules sur lame, avec visualisation de multiples noyaux cellulaires inclus dans un espace délimité par la continuité d'une seule et unique membrane cellulaire. Par ailleurs des photos montrant des cellules en cours de fusion ont permis d'objectiver la réalité de la fusion dès l'analyse en microscopie en contraste de phase et l'absence totale de phénomène équivalent dans les contrôles. Pour objectiver statistiquement l'analyse primaire représentée par les chiffres indiqués dans le tableau 2, un test de Chi-2 a été effectué pour comparer les données du tableau 2.

Les résultats de l'analyse statistique tenant compte du « bruit de fond » de la lecture primaire, sans analyse secondaire après coloration sur lame ou recherche de cellules typiques en cours de fusion qui ne sont jamais vues dans les contrôles sont les suivants :
i) Validation statistique de la spécificité de l'effet pathogène *in vivo :*
   Env exprimée (409) : 20,5 positifs comptés en moyenne sur 100 cellules,
   Env anti-sens (410) : 9,5 positifs comptés en moyenne sur 100 cellules,
   Env mutée (LQMV) : 6,5 positifs comptés en moyenne sur 100 cellules,
   Moyenne des contrôles (410 et LQMV): 9,5 + 6,5/2 = 8%
   Env versus contrôle 410 : Chi-2 = 5,89 (p< 0,02)
   Env versus contrôle LQMV : Chi-2 = 9,83 (p< 0,002)
   Env versus les deux contrôles (410 et LQMV) : Chi-2 = 7,69 (p< 0,01)
   Contrôle 410 versus contrôle LQMV : Chi-2 = 0,61 (Différence Non Significative).

   Les contrôles sont donc bien statistiquement équivalents et il n'y a pas de différence « réelle » liée au type de contrôle.
   Les résultats obtenus dès ce stade de l'analyse (en n'excluant pas le bruit de fond lié aux images artéfactuelles et en comparant entre eux les deux types de contrôles (qui s'avèrent équivalents) est statistiquement très significatif (globalement p<0,01). Les analyses ultérieures, par coloration, de la spécificité des effets ne font donc que confirmer la spécificité de l'effet obtenu *in vivo* en présence de la protéine Env, validant ainsi le modèle animal l'étude *in vivo* de syncytia dont la fusion a été induite par Env HERV-W.
ii) Validation statistique de l'activité thérapeutique des anticorps testés sur l'effet pathogène *in vivo* :
   Env exprimée (409) : 20,5 positifs comptés en moyenne sur 100 cellules,
   Env exprimée (409) + anticorps monoclonal 2H1H8 : 3,5 positifs comptés en moyenne sur 100 cellules.
   Env seule versus injection de l'anticorps 2H1H8 : Chi-2 =15,38 (p <0,001)

Les résultats obtenus montrent un effet statistiquement significatif pour l'anticorps monoclonal (probabilité de résultat dû au hasard (p) inférieure à 0,001). Les analyses ultérieures, par coloration, de la spécificité des effets ne font que confirmer la spécificité de l'effet obtenu *in vivo* en présence de la protéine Env et d'anticorps, validant ainsi l'effet thérapeutique sur le modèle animal.

### Exemple 7 :Etude in vivo de la liaison de la protéine Env HERV-W à des cellules possédant ou ne possédant pas des récepteurs hASCT de type 1 ou 2 et de l'inhibition de cette liaison par injection d'anticorps dirigés contre Env HERV-W.

### 1) Matériel

**Protéine soluble :** surnageant filtré sur 0,45 µm contenant la protéine soluble (cellules 293T tranfectées avec le plasmide 460 (enveloppe-espaceur-His6). Expression vérifiée par Western blot avec un anticorps anti-RGS-His. **L' anticorps :** anticorps monoclonal 2H1H8 (IgG, 5,50mg/ml).

**Les cellules :** XChASCT2, clone cellulaire XC (ATCC CCL-165, cellules de rat) exprimant le récepteur hASCT2.

Milieu DMEM (Gibco Invitrogen 41966-029) avec du sérum Amérique du Sud. Pré-incubation, incubation, marquage en tube Eppendorf 1,5ml.

### 2) Protocole

### 1 Inoculation IP (intrapéritonéale) des cellules XChASCT1, XChASCT2 et de cellules témoins XChASCT- à des souris SCID.

Injection aux souris de 1/5^{ème} de flacon à 70% de confluence sous un volume de 2ml.

### 2 Pré-incubation

Incubation du surnageant protéine soluble (surnageant filtré de la lignée 293T) avec l'anticorps monoclonal 2H1H8 (990 µl de surnageant avec 10µl d'anticorps (dilution au 100^{ème})) pendant 1 heure à 37°C dans l'incubateur des cellules avec une agitation de temps en temps (toutes les 15 minutes).

### 3 Inoculation

Inoculation des protéines seules ou avec l'anticorps, en IP (intrapéritonéal) aux souris greffées avec les cellules (1 10⁶ cellules par point, soit 1/5 d'une boîte de 100 mm de diamètre confluente).

Après injection de l'anticorps (200 microlitres), maintien en IP, pendant 6 heures, avec un massage péritonéal de temps en temps (toutes les 30-60 minutes).

### 4-Récupération des cellules par lavage péritonéal des souris greffées.

- Centrifugation 3000 tours pendant 5 minutes à +4°C.
- Récupération du culot cellulaire et dilution dans les milieux de marquage.(maintien à+4°C jusqu'à fixation.)

### 5 Marquage

- Anticorps primaire :
   Le culot est repris par 100 µl d'anticorps anti-RGS His (dilution au 100^{ème}-Qiagen) dans un tampon PBA (PBS avec 2% sérum de veau foetal et 0,1% azide de sodium), maintenu à +4°C.
   1 heure dans la glace avec une agitation de temps en temps (toutes les 15minutes). lavage en tampon PBA (1 ml par tube), maintenu à +4°C.
- Anticorps secondaire :
   Centrifugation 3000 tours pendant 5 minutes à +4°C.

Le culot est repris par 100µl d'anticorps anti-souris-FITC (dilution au 20^{ème}-DAKO, référence : F0479) dans un tampon PBA), maintenu à +4°C.

1 heure dans la glace avec une agitation de temps en temps (toutes les 15minutes).

2 lavages en tampon PBA (1ml par tube),), maintenu à +4°C.

Culot repris par 500 µl de PBA), maintenu à +4°C. et analyse par FACS. Alternativement analyse par IF après fixation sur lame en acétone/méthanol (50%/50%) à -20°C et contre-coloration au bleu EVANS.

### 3) Souris:

des lots de 2 souris sont inoculés avec :
chaque type de cellules (exprimant les 2 types de récepteurs hASCT1 et hASCT2 et un n'exprimant pas de récepteur hASCT- comme contrôle) sans anticorps (3x2=6 souris)
les trois types de cellules avec la protéine Env et l'anticorps monoclonal 2H1H8 (3x2=6 souris).

### 4) Résultats

Les résultats par lecture en immunofluorescence (IF) au microscope sont présentés dans le tableau 3 ci-dessous:

**Tableau 3**

| Lecture IF: nombre de cellules fluorescentes / nombre de cellules totales (NF / NT) dans un même champ | | |
|---|---|---|
| Lignées | NF / NT | Moyenne |
| XC contrôle | 1/18,0/10 | 1/28 |
| XC + 2H1H8 | 0/20 | 0/20 |
| XChASCT1 contrôle | 12/40, 3/12, 9/25 | 24/77 |
| XChASCT1 +2H1H8 | 1/25, 0/18, 1/30 | 2/73 |
| XChASCT2 contrôle | 8/22, 15/35 | 23/57 |
| XChASCT1 + 2H1H8 | 1/45 | 1/45 |

Chaque chiffre représente le nombre de cellules visualisées comme fluorescentes par champ étudié. Certaines cellules pouvant avoir fixé une fluorescence de manière non spécifique, le compte des cellules apparaissant fluorescentes dans les conditions témoins est donc supérieur à zéro dans un des deux champs compté (moyenne des deux champs = 1/28, soit 0,036%, ce qui est tout à fait correct pour le bruit de fond d'une telle technique de lecture). La réalité des cellules ayant fixé de la protéine Env sur leur récepteur hASCT1 ou hASCT2 a ensuite été vérifiée par analyse cytofluorométrique.

Pour objectiver statistiquement l'analyse présentée dans le tableau 3, un test de Chi-2 a été effectué pour comparer les données relevées dans les conditions selon lesquelles (i) la protéine Env peut se fixer sur un récepteur hASCT1 (hASCT1 contrôle) ou hASCT2 (hASCT2 contrôle) présent à la surface des cellules greffées sur souris SCID versus les cellules contrôles greffées qui n'ont aucun récepteur (X contrôle) sur lesquelles la protéine Env injectée aux animaux correspondants ne peut se fixer et ne donne pas de fluorescence membranaire en présence d'un anticorps anti-Env et (ii) la protéine Env peut se fixer sur un récepteur hASCT1 (hASCT1 contrôle) ou hASCT2 (hASCT2 contrôle) présent à la surface des cellules greffées sur souris SCID versus l'injection d'un anticorps monoclonal dirigé contre Env-SU.

Les résultats de l'analyse statistique sont présentés ci-dessous :
i) Validation statistique de la spécificité de l'effet pathogène *in vivo :*
   hASCT1 contrôle : 24 positifs comptés en moyenne sur 77 cellules,
   hASCT2 contrôle : 23 positifs comptés en moyenne sur 57 cellules,
   cellules hASCT-: 1 positif compté en moyenne sur 28 cellules.
   Env+greffes hASCT1 versus hASCT- : Chi-2 = 8,62 (p< 0.01)
   ENV+greffes hASCT2 versus hASCT- : Chi-2 = 12,53 (p< 0.001)
   Env+greffes hASCT1 versus Env+greffes hASCT2 : Chi-2 = 1,21 (Différence Non Significative).

   Les cellules exprimant les récepteurs hASCT1 ou hASCT2 à leur surface sont donc bien statistiquement équivalentes et il n'y a pas de différence de liaison de Env sur le récepteur liée au sous-type 1 ou 2, dans les conditions de l'expérience.
   Les résultats obtenus avec les animaux greffés avec les cellules exprimant les récepteurs membranaires hASCT1 ou hASCT2 sont statistiquement significatifs au regard des résultats obtenus avec les animaux contrôles greffés avec les cellules n'exprimant aucun de ces récepteurs à leur surface.
   Ces résultats confirment la spécificité de l'effet obtenu *in vivo* en présence de la protéine Env dans les modèles animaux.
ii) Validation statistique de l'activité thérapeutique des anticorps testés sur l'effet pathogène *in vivo :*
   Env+greffes hASCT1 contrôle : 24 positifs comptés en moyenne sur 77 cellules Env+greffes hASCT1 + anticorps monoclonal 2H1H8: 2 positifs comptés en moyenne sur 73 cellules.
   Env+greffes hASCT1 seul versus injection anticorps 2H1H8 : Chi-2 = 21,14(p< 0.001)

Les résultats obtenus montrent un effet statistiquement significatif pour l'anticorps monoclonal (probabilité de résultat dû au hasard (p) inférieure à 0,001).
Env+greffes hASCT2: 23 positifs comptés en moyenne sur 57 cellules
Env+greffes hASCT2 + anticorps monoclonal 2H1H8 : 1 positif compté en moyenne sur 45 cellules.
Env+greffes hASCT2 seul versus injection anticorps 2H1H8 : Chi-2 = 20,31(p< 0.001).

Les résultats obtenus montrent un effet statistiquement significatif pour l'anticorps monoclonal(probabilité de résultat dû au hasard (p) globalement inférieur à 0,01).

La validation des modèles animaux contre les témoins appropriés permet de démontrer que des anticorps peuvent avoir une activité thérapeutique en inhibant significativement les effets pathogènes de la protéine Env HERV-W.

### Exemple 8 : Alignement des séquences du groupe d'interférence

Les séquences protéiques des enveloppes des rétrovirus HERV-W (swiss-prot Q9UQF0), RD114 (swiss-prot Q98654), REV (swiss-prot P31796), BAEV (swiss-prot P10269), SRV1 (swiss-prot P04027), SRV2 (swiss-prot P51515) et MPMV (swiss-prot P07575) ont été alignées à l'aide du logiciel MacVector avec la procédure ClustalW. Le peptide signal, la sous-unité SU (surface unit) et la sous-unité TM (Trans membrane) sont indiquées. Le site de fixation au récepteur est souligné.

## Revendications

1. Domaine peptidique de l'enveloppe d'un virus appartenant au groupe d'interférence de HERV-W, capable d'interagir avec un récepteur hASCT, défini en ce qu'il commence par une extrémité N terminale et se termine par une extrémité C terminale, et en ce que :
l'extrémité N-terminale est définie par un motif choisi parmi SEQ ID N°1 à SEQ ID N°29,
l'extrémité C terminale est définie par un motif choisi parmi SEQ ID N°30 à SEQ ID N°40,
et en ce que ledit domaine peptidique comprend, entre l'extrémité N terminale et l'extrémité C terminale au moins un motif choisi parmi SEQ ID N°41, SEQ ID N°42 et SEQ ID NO: 73.

2. Domaine peptidique tel que défini dans la revendication 1, **caractérisé en ce qu'**il induit une réponse immune contre un virus appartenant au groupe d'interférence de HERV-W et **en ce qu'**il consiste en SEQ ID NO : 74 illustrée à la Figure 3a.

3. Anticorps monoclonal dirigé contre un domaine peptidique tel que défini dans la revendication 2.

4. Utilisation d'au moins un domaine peptidique tel que défini dans la revendication 1 ou 2, ou d'au moins un anticorps tel que défini dans la revendication 3, pour la préparation d'un médicament destiné à l'inhibition, la prévention ou le traitement d'une infection provoquée par un virus appartenant au groupe d'interférence de HERV-W chez un animal, de préférence l'homme.

5. Composition pharmaceutique comprenant à titre de substance active au moins un domaine peptidique tel que défini dans la revendication 1 ou 2, ou bien au moins un anticorps tel que défini dans la revendication 3, en association avec un véhicule pharmaceutiquement approprié.

6. Composition diagnostique pour la détection et/ou la quantification d'un virus appartenant au groupe d'interférence de HERV-W et/ou la quantification d'une réponse immune contre un virus appartenant au groupe d'interférence de HERV-W, comprenant au moins un domaine peptidique tel que défini dans la revendication 1 ou 2, ou au moins un anticorps tel que défini dans la revendication 3.

7. Procédé de détection et/ou de quantification d'un virus appartenant au groupe d'interférence de HERV-W dans un échantillon biologique prélevé chez un individu susceptible d'être infecté par ledit virus **caractérisé en ce qu'**il comprend les étapes consistant à :
- mettre en contact ledit échantillon biologique avec au moins un anticorps selon la revendication 3 dans des conditions permettant la formation d'un complexe entre le virus et l'anticorps, et
- détecter et/ou quantifier la formation dudit complexe par tout moyen approprié.

8. Utilisation de la composition selon la revendication 6 pour le diagnostic *in vitro* d'un virus appartenant au groupe d'interférence de HERV-W dans un échantillon ou prélèvement biologique.

9. Utilisation d'un domaine peptidique selon la revendication 1 pour identifier des molécules chimiques ou biologiques dont l'interaction avec tout ou partie de ce domaine peptidique, bloque l'interaction entre l'enveloppe d'un virus appartenant au groupe d'interférence de HERV-W et un récepteur hASCT.

## Patentansprüche

1. Peptiddomäne der Hülle eines der HERV-W-Interferenzgruppe angehörenden Virus, die in der Lage ist, mit einem hASCT-Rezeptor zu interagieren, dadurch definiert, dass sie mit einem N-Terminus beginnt und mit einem C-Terminus endet, und dadurch, dass:
der N-Terminus definiert ist durch ein Motiv, ausgewählt aus SEQ-ID Nr. 1 bis SEQ-ID Nr. 29,
der C-Terminus definiert ist durch ein Motiv, ausgewählt aus SEQ-ID Nr. 30 bis SEQ-ID Nr. 40,
und dadurch, dass die besagte Peptiddomäne zwischen dem N-Terminus und dem C-Terminus mindestens ein Motiv, ausgewählt aus SEQ-ID Nr. 41, SEQ-ID Nr. 42 und SEQ-ID Nr. 73 umfasst.

2. Peptiddomäne wie in Anspruch 1 definiert, **dadurch gekennzeichnet, dass** sie eine Immunantwort gegen ein der HERV-W-Interferenzgruppe angehörendes Virus induziert und dadurch, dass sie aus der in Figur 3a dargestellten SEQ-ID Nr. 74 besteht.

3. Monoklonaler Antikörper, der gegen eine Peptiddomäne wie in Anspruch 2 definiert gerichtet ist.

4. Verwendung mindestens einer Peptiddomäne wie in Anspruch 1 oder 2 definiert, oder mindestens eines Antikörpers wie in Anspruch 3 definiert, zur Herstellung eines Arzneimittels, das zum Hemmen, Vorbeugen oder Behandeln einer durch ein der HERV-W-Interferenzgruppe angehörendes Virus hervorgerufenen Infektion bei einem Tier, bevorzugt dem Menschen, bestimmt ist.

5. Pharmazeutische Zusammensetzung, die als Wirkstoff mindestens eine Peptiddomäne wie in Anspruch 1 oder 2 definiert, oder mindestens einen Antikörper wie in Anspruch 3 definiert, in Kombination mit einem pharmazeutisch geeigneten Träger umfasst.

6. Diagnostische Zusammensetzung zum Nachweis und/oder zur Quantifizierung eines der HERV-W-Interferenzgruppe angehörenden Virus und/oder zur Quantifizierung einer Immunantwort gegen ein der HERV-W-Interferenzgruppe angehörendes Virus, die mindestens eine Peptiddomäne wie in Anspruch 1 oder 2 definiert, oder mindestens einen Antikörper wie in Anspruch 3 definiert umfasst.

7. Verfahren zum Nachweis und/oder zur Quantifizierung eines der HERV-W-Interferenzgruppe angehörenden Virus in einer bei einem vermutlich mit dem besagten Virus infizierten Individuum entnommenen biologischen Probe, **dadurch gekennzeichnet, dass** es die Schritte umfasst bestehend aus:
- Inkontaktbringen der besagten biologischen Probe mit mindestens einem Antikörper nach Anspruch 3 unter Bedingungen, die die Bildung eines Komplexes zwischen dem Virus und dem Antikörper gestatten, und
- Nachweisen und/oder Quantifizieren der Bildung des besagten Komplexes mit allen geeigneten Mitteln.

8. Verwendung der Zusammensetzung nach Anspruch 6 zur In-vitro-Diagnose eines der HERV-W-Interferenzgruppe angehörenden Virus in einer biologischen Probe oder Entnahme.

9. Verwendung einer Peptiddomäne nach Anspruch 1 zum Identifizieren von chemischen oder biologischen Molekülen, deren Interaktion mit der gesamten oder einem Teil dieser Peptiddomäne die Interaktion zwischen der Hülle eines der HERV-W-Interferenzgruppe angehörenden Virus und einem hASCT-Rezeptor blockiert.

## Claims

1. A peptide domain of the envelope of a virus belonging to the HERV-W interference group, capable of interacting with a hASCT receptor, defined in that it starts with an N-terminus and ends with a C-terminus, and in that:
the N-terminus is defined by a motif selected from SEQ ID NO°1 to SEQ ID NO°29,
the C-terminus is defined by a motif selected from SEQ ID NO°30 to SEQ ID NO°40,
and in that said peptide domain comprises, between the N-terminus and the C-terminus, at least one motif selected from SEQ ID NO°41, SEQ ID NO°42 and SEQ ID NO:73.

2. The peptide domain as defined in claim 1, **characterized in that** it induces an immune response against a virus belonging to the HERV-W interference group and **in that** it consists of SEQ ID NO: 74 illustrated in figure 3a.

3. A monoclonal antibody directed against a peptide domain as defined in claim 2.

4. A use of at least one peptide domain as defined in claim 1 or 2, or of at least one antibody as defined in claim 3, for preparing a drug intended to inhibit, prevent or treat an infection caused by a virus belonging to the HERV-W interference group in an animal, preferably in humans.

5. A pharmaceutical composition comprising, as an active substance, at least one peptide domain as defined in claim 1 or 2, or at least one antibody as defined in claim 3, in association with a pharmaceutically suitable vehicle.

6. A diagnostic composition for the detection and/or quantification of a virus belonging to the HERV-W interference group and/or the quantification of an immune response against a virus belonging to the HERV-W interference group, comprising at least one peptide domain as defined in claim 1 or 2, or at least one antibody as defined in claim 3.

7. A method for detecting and/or quantifying a virus belonging to the HERV-W interference group in a biological sample taken from an individual likely to be infected with said virus **characterized in that** it comprises the steps consisting in:
- contacting said biological sample with at least one antibody according to claim 3 under conditions allowing the formation of a complex between the virus and the antibody, and
- detecting and/or quantifying the formation of said complex by any appropriate means.

8. A use of the composition according to claim 6 for the *in vitro* diagnosis of a virus belonging to the HERV-W interference group in a biological sample or collection.

9. A use of a peptide domain according to claim 1 for identifying chemical or biological molecules whose interaction with all or part of this peptide domain, blocks the interaction between the envelope of a virus belonging to the HERV-W interference group and a hASCT receptor.
